# EUROPÄISCHE PATENTSCHRIFT

(11) **EP 3 509 994 B1**
(45) Veröffentlichungstag und Bekanntmachung des Hinweises auf die Patenterteilung: **19.06.2024**
(21) Anmeldenummer: 17771688.3
(22) Anmeldetag: 07.09.2017
(51) Int. Cl.: C01F 11/18, A61L 27/00, B01J 2/00, C08J 3/12, C08J 3/20, C09C 1/00, C09C 1/02, C08L 67/04

(54) **CALCIUMCARBONAT-ENTHALTENDES VERBUNDPULVER MIT MIKROSTRUKTURIERTEN TEILCHEN**
CALCIUM CARBONATE-CONTAINING COMPOSITE POWDER WITH MICROSTRUCTURED PARTICLES
POUDRE COMPOSITE CONTENANT DU CARBONATE DE CALCIUM À PARTICULES MICROSTRUCTURÉES

(30) Priorität: 08.09.2016 EP 16001954
(43) Veröffentlichungstag der Anmeldung: 17.07.2019
(73) Patentinhaber: Schaefer Kalk GmbH & Co. KG, 65582 Diez (DE)
(72) Erfinder: VUCAK, Marijan, 65624 Altendiez (DE)
(74) Vertreter: Mai Besier
(86) Internationale Anmeldenummer: PCT/EP2017/072412
(87) Internationale Veröffentlichungsnummer: WO 2018/046572

(56) Entgegenhaltungen:
- WO-A2-2012/013349
- WO-A2-2012/126600
- US-A- 4 915 884
- US-A- 5 011 862

## Beschreibung

Calciumcarbonat-enthaltendes Verbundpulver mit mikrostrukturierten Teilchen Die vorliegende Erfindung betrifft ein Calciumcarbonat-enthaltendes Verbundpulver mit mikrostrukturierten Teilchen, seine Verwendung sowie Bauteile, erhältlich durch selektives Lasersintern, ausgenommen Implantate für Anwendungen im Bereich der Neuro-, Mund-, Kiefer-, Gesichts-, Hals-, Nasen- und Ohrenchirurgie sowie der Hand-, Fuß-, Thorax-, Rippen- und Schulterchirurgie.

Calciumcarbonat, CaCOs, ist ein Calcium-Salz der Kohlensäure, das heutzutage in vielen Bereichen des täglichen Lebens Anwendung findet. So wird es insbesondere als Additiv oder Modifizierungsmittel in Papier, Farben, Kunststoffen, Tinten, Klebstoffen und Pharmazeutika eingesetzt. In Kunststoffen dient Calciumcarbonat vorrangig als Füllstoff, um das vergleichsweise teure Polymer zu ersetzen.

Auch Verbundwerkstoffe (Kompositwerkstoffe) sind bereits bekannt und bezeichnen einen Werkstoff aus zwei oder mehr verbundenen Materialien, der andere Werkstoffeigenschaften als seine einzelnen Komponenten besitzt. Für die Eigenschaften der Verbundwerkstoffe sind die stofflichen Eigenschaften und die Geometrie der Komponenten von Bedeutung. Insbesondere spielen oft Größeneffekte eine Rolle. Die Verbindung erfolgt in der Regel durch Stoff- oder Formschluss oder eine Kombination von beidem.

Weiterhin sind auch mikrostrukturierte Verbundteilchen, enthaltend Calciumsalze, insbesondere Calciumcarbonat, an sich schon bekannt.

So offenbart WO 2012/126600 A2 mikrostruktierte Verbundteilchen, erhältlich durch ein Verfahren, bei welchem man große Teilchen mit kleinen Teilchen verbindet, wobei
- die großen Teilchen einen mittleren Teilchendurchmesser im Bereich von 0,1 µm bis 10 mm aufweisen,
- der mittlere Teilchendurchmesser der kleinen Teilchen höchstens 1/10 des mittleren Teilchendurchmessers der großen Teilchen ist,
- die großen Teilchen mindestens ein Polymer umfassen,
- die kleinen Teilchen Calciumcarbonat umfassen,
- die kleinen Teilchen auf der Oberfläche der großen Teilchen angeordnet und/oder inhomogen innerhalb der großen Teilchen verteilt sind,
wobei die kleinen Teilchen gefällte Calciumcarbonat-Teilchen mit einer mittleren Teilchengröße im Bereich von 0,01 µm bis 1,0 mm umfassen.

Weiterhin beschreibt WO 2012/126600 A2 mikrostruktierte Verbundteilchen, erhältlich durch ein Verfahren, bei welchem man große Teilchen mit kleinen Teilchen verbindet, wobei
- die großen Teilchen einen mittleren Teilchendurchmesser im Bereich von 0,1 µm bis 10 mm aufweisen,
- der mittlere Teilchendurchmesser der kleinen Teilchen höchstens 1/10 des mittleren Teilchendurchmessers der großen Teilchen ist,
- die großen Teilchen mindestens ein Polymer umfassen,
- die kleinen Teilchen mindestens ein Calciumsalz umfassen,
- die kleinen Teilchen auf der Oberfläche der großen Teilchen angeordnet und/oder inhomogen innerhalb der großen Teilchen verteilt sind,
wobei die großen Teilchen mindestens einen resorbierbaren Polyester mit einem Zahlenmittel des Molekulargewichts im Bereich von 500 g/mol bis 1.000.000 g/mol umfassen.

Die in der WO 2012/126600 A2 gezeigten Verbundteilchen sollen sich vor allem als Additiv, insbesondere als Polymeradditiv, als Zusatzsatzstoff oder Ausgangsmaterial für die Herstellung von Bauteilen, für Anwendungen in der Medizintechnik und/oder in der Mikrotechnik und/oder für die Herstellung von geschäumten Gegenständen eignen. Das Verfahren des selektiven Lasersintern (SLM-Verfahren) wird in der Druckschrift u. a. erwähnt.

Allerdings sind besser geeignete Materialien für selektives Lasersintern wünschenswert. Ein Nachteil der Verbundteilchen der WO 2012/126600 A2 ist insbesondere ihre schlechte Rieselfähigkeit, die auch durch die Verwendung von Rieselhilfsmitteln nur teilweise reduziert werden kann. Vor allem für die Herstellung von Implantaten sind Zusätze von derartigen Rieselhilfsmitteln nicht vorteilhaft, da sie in der Regel die Eigenschaften des resultierenden Implantats, insbesondere seine Bioverträglichkeit und Bioabbaubarkeit, nachteilig beeinflussen. Weiterhin ist wegen der schlechten Rieselfähigkeit der Transport in der Lasersinteranlage erschwert.

Bei der Herstellung von Bauteilen durch Lasersintern unter Verwendung der Materialien der WO 2012/126600 A2 treten folgende zusätzliche Probleme auf. Die Sinterung von gemahlenen Verbundteilchen ist zwar durchführbar, aber die Oberflächengüte und Oberflächenbeschaffenheit sowie die Bauteildichte der resultierenden Bauteile ist nicht voll befriedigend. Wünschenswert wären insbesondere ein besseres Schrumpfverhalten und eine verbesserte Dimensionsstabilität der resultierenden Bauteile sowie ein besseres Wärmeleitverhalten außerhalb des laserbehandelten Bereichs. Darüber hinaus wäre ein effizienteres Herstellungsverfahren von Bauteilen wünschenswert.

US 4 915 884 A betrifft ein Verfahren zur Herstellung eines granularen Materials für die Wasserbehandlung, welches die Herstellung von Granulaten mit einer spezifischen Dichte d und einer Größe t ermöglicht, die unabhängig voneinander im Bereich von 1<d≤3 und 0,5 mm≤t≤10 mm eingestellt werden können, um die Granulate an die Behandlungsart, für die das Material eingesetzt werden soll, anpassen zu können. Das Verfahren umfasst die Bildung einer Mischung aus einem oxidierbaren thermoplastischen Harz mit einer spezifischen Dichte dr kleiner d und einem Hilfsmittel in Form eines Pulvers mit einer Granulometrie kleiner als ungefähr 200 µm und einer spezifischen Dichte da, die die Vorgabe da>1 ,7d-0,7dr erfüllt, wobei der Gewichtsanteil des Hilfsmittels pa eine spezielle Vorgabe erfüllen soll. Die Mischung wird in den plastischen Zustand erhitzt, zu zylindrischen Strängen extrudiert, die sofort in Plastikstücke geschnitten werden, die kürzer als ihr Durchmesser sind. Die Plastikstücke werden zu sphärischen Granulaten verfestigt und an der Oberfläche einer Oxidation zur Bildung hydrophiler Stellen unterzogen.

Ggf. wird ein kationisches Polyelektrolyt auf die Oberfläche der Granulate aufgepfropft.

Als thermoplastisches Harz wird beispielsweise HDPE eingesetzt.

Als Hilfsmittel wird beispielsweise Calciumcarbonat mit einer mittleren Teilchengröße von 30 µm und einer spezifischen Dichte von 2,71 eingesetzt.

Die Oxidation erfolgt beispielsweise mit einer Mischung aus Schwefelsäure und Kaliumdichromat.

Jedoch ist dieser Druckschrift kein Hinweis auf kugelförmige Calciumcarbonat-Teilchen zu entnehmen.

Vor diesem Hintergrund lag der vorliegenden Erfindung die Aufgabe zugrunde, Möglichkeiten zur Bereitstellung eines Calciumcarbonat-enthaltenden Verbundpulvers mit verbesserten Eigenschaften aufzuzeigen. Insbesondere sollte ein Material mit verbesserten Lasersintereigenschaften bereitgestellt werden, welches insbesondere eine verbesserte Rieselfähigkeit besitzt, beim Lasersintern die Herstellung von Bauteilen mit verbesserter Oberflächengüte und Oberflächenbeschaffenheit sowie verbesserter Bauteildichte ermöglicht und insbesondere besseres Schrumpfverhalten und eine verbesserte Dimensionsstabilität der resultierenden Bauteile sowie ein besseres Wärmeleitverhalten außerhalb des laserbehandelten Bereichs zeigt. Zusätzlich wurde ein effizienteres Herstellungsverfahren von Bauteilen gewünscht. Schließlich war auch die Bereitstellung besonders vorteilhafter Bauteile Ziel der vorliegenden Erfindung. Darüber hinaus wurden lösungsmittelfreie Produkte angestrebt, die insbesondere in Bereichen mit restriktiven Vorgaben bzgl. der Anwesenheit von Lösungsmittelresten im Produkt problemlos eingesetzt werden können. Besonders hervorzuheben sind in diesem Zusammenhang Produkte für medizintechnische Anwendungen, die in der Regel vollkommen lösungsmittel frei sein müssen. Schließlich wurden auch Möglichkeiten gesucht, thermischen Abbau, insbesondere Polymerdegradation während der Herstellung der Endprodukte bestmöglich zu verhindern.

Gelöst werden diese sowie weitere nicht konkret genannten Aufgaben, die sich aus den obigen Zusammenhängen direkt ableiten lassen, durch die Bereitstellung eines Verbundpulvers mit mikrostrukturierten Teilchen mit allen Merkmalen des vorliegenden Anspruchs 1. Die auf Anspruch 1 rückbezogenen Unteransprüche beschreiben besonders zweckmäßige Varianten des Verbundpulvers. Der Verwendungsanspruch betrifft eine besonders zweckmäßige Anwendung des erfindungsgemäßen Verbundpulvers. Weiterhin wird ein besonders vorteilhaftes Bauteil unter Schutz gestellt, das durch selektives Lasersintern einer Zusammensetzung erhältlich ist, welche ein erfindungsgemäßes Verbundpulver enthält, ausgenommen Implantate für Anwendungen im Bereich der Neuro-, Mund-, Kiefer-, Gesichts-, Hals-, Nasen- und Ohrenchirurgie sowie der Hand-, Fuß-, Thorax-, Rippen- und Schulterchirurgie. Gegenstand der Erfindung sind auch die kugelförmigen Calciumcarbonat-Teilchen, die zur Herstellung der erfindungsgemäßen Verbundteilchen vorteilhaft eingesetzt werden können sowie deren Verwendung.

Durch die Bereitstellung eines Verbundpulvers mit mikrostrukturierten Teilchen, erhältlich durch ein Verfahren, bei welchem man große Teilchen mit kleinen Teilchen verbindet, wobei
- die großen Teilchen einen mittleren Teilchendurchmesser im Bereich von 0,1 µm bis 10 mm aufweisen,
- die großen Teilchen mindestens ein Polymer umfassen,
- die kleinen Teilchen auf der Oberfläche der großen Teilchen angeordnet und/oder inhomogen innerhalb der großen Teilchen verteilt sind,
- die kleinen Teilchen kugelförmige gefällte Calciumcarbonat-Teilchen mit einem mittleren Durchmesser im Bereich von 0,05 µm bis 50,0 µm, bevorzugt im Bereich von 2,5 µm bis 30,0 µm, umfassen,
wobei die kugelförmigen Calciumcarbonat-Teilchen nach einem Verfahren erhältlich sind, bei welchem man
a. eine Calciumhydroxid-Suspension vorlegt,
b. in die Suspension aus Schritt a. Kohlendioxid oder eine Kohlendioxid-haltige Gasmischung einleitet und
c. entstehende Calciumcarbonat-Teilchen abtrennt,
wobei man weiterhin 0,3 Gew.-% bis 0,7 Gew.-% mindestens einer Aminotrialkylenphosphonsäure zugibt, gelingt es auf nicht ohne weiteres vorhersehbare Weise ein Calciumcarbonat-enthaltendes Verbundpulver mit verbesserten Eigenschaften zugänglich zu machen, welche insbesondere zur Anwendung in Lasersinterverfahren hervorragend geeignet sind. Das erfindungsgemäße Verbundpulver besitzt eine verbesserte Rieselfähigkeit, ermöglicht beim Lasersintern die Herstellung von Bauteilen mit verbesserter Oberflächengüte und Oberflächenbeschaffenheit sowie verbesserter Bauteildichte. Gleichzeitig zeigen die resultierenden Bauteile insbesondere ein besseres Schrumpfverhalten und eine verbesserte Dimensionsstabilität. Weiterhin ist ein besseres Wärmeleitverhalten außerhalb des laserbehandelten Bereichs festzustellen.

Darüber hinaus erlaubt das erfindungsgemäße Verbundpulver eine effizientere Herstellung von Bauteilen, insbesondere nach dem Lasersinterverfahren. Der Schmelzefluß der unter Verwendung des erfindungsgemäßen Verbundpulvers erhältlichen Schmelze ist deutlich erhöht (verbessert). Das erfindungsgemäße Verbundpulver ist im Vergleich mit herkömmlichen Materialien insbesondere nach dem SLM-Verfahren besser verarbeitbar und ermöglicht einen deutlich besseren Schichtaufbau im SLM-Verfahren. Die unter Verwendung des erfindungsgemäßen Verbundpulvers nach dem SLM Verfahren erhältlichen Bauteile zeichnen sich durch eine extrem hohe Qualität aus und weisen im Vergleich mit Bauteilen, die unter Verwendung herkömmlicher Materialien nach dem SLM Verfahren hergestellt wurden, deutlich weniger Fehlstellen, eine höhere Bauteildichte, vorzugsweise größer 95%, insbesondere größer 97%, sowie eine geringere Porosität auf. Gleichzeitig ist der Gehalt an Abbauprodukten in den resultierenden Bauteilen deutlich geringer und die Zellverträglichkeit der Bauteile extrem hoch.

Auch die übrigen Eigenschaften der auf diese Weise erhältlichen Bauteile ist exzellent. Die Bauteile weisen sehr gute mechanische Eigenschaften sowie eine sehr gute pH-Stabilität auf. Gleichzeitig ist die Bioverträglichkeit der Bauteile deutlich verbessert. Vergleichbare Bauteile sind unter Verwendung der reinen Polymere nicht erhältlich, zumal entsprechende Polymerpulver, die nach dem SLM-Verfahren verarbeitet werden könnten, nicht bekannt sind.

Ein weiterer Vorteil der vorliegenden Erfindung ist darin zu sehen, dass die Eigenschaften des erfindungsgemäßen Verbundpulvers, insbesondere die Fließeigenschaften des Verbundpulvers, durch die Einsatzmengen und die Eigenschaften der großen Teilchen und der kleinen Teilchen, insbesondere durch die Eigenschaften der Calciumcarbonat-Teilchen, vor allem durch die Partikelgröße der Calciumcarbonat-Teilchen, sowie durch die Menge der Calciumcarbonat-Teilchen gezielt gesteuert und eingestellt werden können. Darüber hinaus können durch Klassierung des Verbundpulvers insbesondere der Calciumcarbonat-Gehalt des Verbundpulvers und die Fließeigenschaften des Verbundpulvers verändert und dem jeweiligen Anwendungszweck gezielt angepasst werden.

Insbesondere in Kombination mit Polylactid als Polymer ergeben sich erfindungsgemäß die folgenden Vorteile.

Unter Verwendung des erfindungsgemäßen Verbundpulvers können abbaubare Implantate mit steuerbarer Resorptionskinetik und einstellbaren mechanischen Eigenschaften erzeugt werden. Polylactide, welche vorzugsweise im Verbundpulver enthalten sind, sind biodegradierbare Polymere auf Basis von Milchsäure. Im Organismus werden Polylactide durch Hydrolyse abgebaut. Calciumsalze, insbesondere Calciumphosphat und Calciumcarbonat, sind mineralische Werkstoffe auf Basis von Calcium und werden im Körper durch den natürlichen Regenerationsprozess des Knochens abgebaut. Calciumcarbonat besitzt die besonders vorteilhafte Eigenschaft, das für Knochenzellen mitunter toxische acide Milieu beim Abbau der Polylactide zu puffern. Im Vergleich zu Calciumphosphat (pH 4) puffert Calciumcarbonat bereits bei einem pH-Wert von ca. 7, d. h. nahe dem physiologischen Wert von 7,4. Über die Molekülkettenlänge und die chemische Zusammensetzung des Polymers, insbesondere des Polylactides, kann die Zeit bis zur vollständigen Degradation angepasst werden. Ähnliches ist für die mechanischen Eigenschaften des Polymers möglich.

Das erfindungsgemäße Verbundpulver kann mit Hilfe des generativen Fertigungsverfahrens Selective Laser Melting (SLM) zu Implantatstrukturen verarbeitet werden. Hierbei ist eine gezielte Anpassung von Werkstoff und Fertigungsverfahren aneinander und an die medizinischen Erfordernisse möglich. Die Nutzung der generativen Fertigung und der damit einhergehenden Geometriefreiheit bietet die Möglichkeit, das Implantat mit einer den Wünschen des Chirurgen entsprechenden, inneren und offenen Porenstruktur zu versehen, die eine durchgängige Versorgung des Implantates gewährleistet. Darüber hinaus können generativ individuell angepasste Implantate, wie für die Versorgung von großflächigen Knochendefekten im Gesichts- und Schädelbereich benötigt, schnell und wirtschaftlich hergestellt werden. Der Vorteil des erfindungsgemäßen Verbundpulvers für die Verarbeitung mittels SLM besteht insbesondere darin, dass das Polymer durch die Laserstrahlung bei relativ niedrigen Temperaturen, bevorzugt kleiner 300 °C, geschmolzen werden kann und die Calciumcarbonat-Teilchen bei diesen Temperaturen thermisch stabil bleiben. Durch maßgeschneiderte Synthese des erfindungsgemäßen Verbundpulvers können somit die Calciumcarbonat-Teilchen ohne thermische Schädigung durch die Laserstrahlung homogen im gesamten Volumen des Implantats in einer Matrix aus Polylactid eingebettet werden. Die Festigkeit des Implantats wird zum einen durch die Polylactid-Matrix bestimmt und zum anderen durch die Morphologie der Calciumcarbonat-Teilchen sowie bevorzugt auch durch das Mischungsverhältnis der verwendeten Komponenten. Die Implantate sind zudem bioaktiv, da sie über die Werkstoffauswahl und die anschließende Beschichtung mit einem wachstumsstimulierenden Protein (rhBMP-2) das umgebende Knochengewebe aktiv zum Knochenaufbau und Ersatz der Gerüststruktur (Implantat) anregen.

Die wesentlichen Vorteile der mittels SLM generativ gefertigten Implantate aus dem erfindungsgemäßen Verbundpulver sind insbesondere:
∘ Mit der Verwendung biodegradierbarer, osteokonduktiver Werkstoffe wird das Durchwachsen des Implantats mit Knochen aktiv stimuliert und auch bei großflächigen Defekten die komplette Degradation bei vollständiger Knochenneubildung im zu versorgenden Knochendefekt erreicht. Durch die interkonnektierende Porenstruktur kann die BMP-Beschichtung im gesamten "Volumen" des Implantats aktiv wirken.
∘ Einsprossung Knochengewebe: Das Einbringen einer geeigneten Porenstruktur begünstigt das Einsprossen neuen Knochengewebes in das Implantat. Mit dem generativen Fertigungsprozess kann eine definierte Porenstruktur reproduzierbar in die Bauteile eingebracht werden.
∘ Die vorgeschlagene Lösung bietet weiterhin den Vorteil, medizinische Komplikationen von Langzeitimplantaten bestmöglich zu verhindern, das Wohlbefinden des Patienten durch die Vermeidung eines permanenten Fremdkörpergefühls bestmöglich zu steigern und - vor allem bei Kindern und Jugendlichen - ein "mitwachsendes" Implantat bestmöglich zu realisieren.
∘ Optimale Pufferung: Durch die Verwendung von Calciumcarbonat wird die acide Degradation des Werkstoffs Polylactid bereits bei einem pH-Wert von ca. 7 gepuffert, so dass das entstehende acide Milieu in der Umgebung des Implantats und damit eine inflammatorische oder zytotoxische Wirkung vermieden werden kann. Darüber hinaus werden Degradationsprozesse des Polymers, insbesondere des Milchsäurepolymers, bestmöglich unterdrückt.
∘ Große Festigkeit: Durch den SLM Prozess wird ein vollständiger Schmelzverbund und damit eine große Bauteildichte und -festigkeit erzeugt, wodurch auch großflächige Defekte mit individuell angepassten Implantate aus einem biodegradierbaren Werkstoff und offener Porenstruktur versorgt werden können.

Darüber hinaus können die erfindungsgemäßen Produkte ohne die Verwendung von herkömmlichen Lösungsmitteln hergestellt werden und zeichnen sich daher vorzugsweise durch diese "Lösungsmittelfreiheit" aus. Dies ermöglicht ihren Einsatz insbesondere in Bereichen mit restriktiven Vorgaben bzgl. der Anwesenheit von Lösungsmittelresten im Produkt, da hier die erfindungsgemäßen Produkte problemlos eingesetzt werden können. Besonders hervorzuheben sind in diesem Zusammenhang medizintechnische Anwendungen, die in der Regel vollkommen lösungsmittel frei sein müssen. Schließlich kann das erfindungsgemäße Verbundpulver auf vergleichsweise einfache Art und Weise zu den gewünschten Endprodukten weiterverarbeitet werden. Ein thermischer Abbau, insbesondere Polymerdegradation während der Herstellung der Endprodukte wird bestmöglich verhindert.

Gegenstand der vorliegenden Erfindung sind dementsprechend Verbundpulver mit mikrostrukturierten Teilchen (Kompositpulver), das durch ein Verfahren erhältlich sind, bei welchem man große Teilchen mit kleinen Teilchen verbindet.

Als Mikrostruktur werden in der vorliegenden Erfindung die mikroskopischen Eigenschaften eines Materials bezeichnet. Dazu gehören unter anderem die auflösbare Feinstruktur und das Gefüge. Bei Flüssigkeiten sowie Gasen sind diese nicht vorhanden. Hier befinden sich die einzelnen Atome oder Moleküle in einem ungeordneten Zustand. Amorphe Festkörper weisen zumeist eine strukturelle Nahordnung im Bereich der Nachbaratome auf, jedoch keine Fernordnung. Kristalline Festkörper hingegen weisen nicht nur im Nahbereich, sondern auch im Fernbereich eine geordnete Gitterstruktur auf.

Im Rahmen der vorliegenden Erfindung umfassen die großen Teilchen mindestens ein Polymer, welches grundsätzlich keinen weiteren Beschränkungen unterliegt. Es handelt sich jedoch bevorzugt um ein thermoplastisches Polymer, zweckmäßigerweise ein Biopolymer, ein Kautschuk, insbesondere Naturkautschuk oder Synthesekautschuk, und/oder ein Polyurethan.

Der Begriff *"thermoplastisches Polymer"* bezeichnet in diesem Zusammenhang einen Kunststoff, der sich in einem bestimmten Temperaturbereich, bevorzugt im Bereich von 25°C bis 350°C, (thermoplastisch) verformen lässt. Dieser Vorgang ist reversibel, das heißt er kann durch Abkühlung und Wiedererwärmung bis in den schmelzflüssigen Zustand beliebig oft wiederholt werden, solange nicht durch Überhitzung die sogenannte thermische Zersetzung des Materials einsetzt. Darin unterscheiden sich thermoplastische Polymere von den Duroplasten und Elastomeren.

Der Begriff *"Biopolymer"* bezeichnet einen Werkstoff, der aus biogenen Rohstoffen (nachwachsenden Rohstoffen) besteht und/oder biologisch abbaubar ist (biogenes und/oder biologisch abbaubares Polymer). Unter diesen Begriff fallen also biobasierte Biopolymere, die biologisch abbaubar oder auch nicht biologisch abbaubar sind, sowie erdölbasierte Polymere, die biologisch abbaubar sind. Damit erfolgt eine Abgrenzung von den konventionellen, erdölbasierten Werkstoffen bzw. Kunststoffen, die nicht biologisch abbaubar sind, wie z. B. Polyethylen (PE), Polypropylen (PP) und Polyvinylchlorid (PVC).

Der Begriff *"Kautschuk"* bezeichnet ein hochmolekulares, unvernetztes polymeres Material mit gummi-elastischen Eigenschaften bei Raumtemperatur (25°C). Bei höheren Temperaturen oder unter Einfluss von Verformungskräften zeigt ein Kautschuk ein zunehmendes viskoses Fließen und ermöglicht so sein Umformen unter geeigneten Bedingungen.

Gummi-elastisches Verhalten ist durch einen relativ geringen Schermodul mit einer eher geringen Temperaturabhängigkeit gekennzeichnet. Es wird durch Entropieänderungen verursacht. Durch Verstrecken wird das gummi-elastische Material in eine geordnetere Konfiguration gezwungen, die zu einer Entropieabnahme führt. Nach Entfernung der Kraft kehren die Polymere daher wieder in ihre Ursprungsposition zurück und die Entropie erhöht sich wieder.

Der Begriff *"Polyurethan"* (PU, DIN-Kurzzeichen: PUR) bezeichnet einen Kunststoff oder ein Kunstharz, welcher oder welches aus der Polyadditionsreaktion von Diolen oder Polyolen mit Polyisocyanaten entsteht. Charakteristisch für ein Polyurethan ist die Urethan-Gruppe.

Im Rahmen der vorliegenden Erfindung werden thermoplastische Polymere besonders bevorzugt eingesetzt. Besonders geeignete Polymere schließen dabei die folgenden Polymere ein: Acrylnitril-Ethylen-Propylen-(Dien)-Styrol-Copolymer, Acrylnitril-Methacrylat-Copolymer, Acrylnitril-Methylmethacrylat-Copolymer, Acrylnitril-chloriertes Polyethylen-Styrol-Copolymer, Acrylnitril-Butadien-Styrol-Copolymer, Acrylnitril-Ethylen-Propylen-Styrol-Copolymer, aromatische Polyester, Acrylnitril-Styrol-Acrylester-Copolymer, Butadien-Styrol-Copolymer, Celluloseacetat, Celluloseacetobutyrat, Celluloseacetopropionat, hydrierte Cellulose, Carboxymethylcellulose, Cellulosenitrat, Cellulosepropionat, Cellulosetriacetat, Polyvinylchlorid, Ethylen-Acrylsäure-Copolymer, Ethylen-Butylacrylat-Copolymer, Ethylen-Chlortrifluorethylen-Copolymer, Ethylen-Ethylacrylat-Copolymer, Ethylen-Methacryat-Copolymer, Ethylen-Methacrylsäure-Copolymer, Ethylen-Tetrafluorethylen-Copolymer, Ethylen-Vinylalkohol-Copolymer, Ethylen-Buten-Copolymer, Ethylcellulose, Polystyrol, Polyfluorethylenpropylen, Methylmethacrylat-Acrylnitril-Butadien-Styrol-Copolymer, Methylmethacrylat-Butadien-Styrol-Copolymer, Methylcellulose, Polyamid 11, Polyamid 12, Polyamid 46, Polyamid 6, Polyamid 6-3-T, Polyamid 6-Terephthalsäure-Copolymer, Polyamid 66, Polyamid 69, Polyamid 610, Polyamid 612, Polyamid 6I, Polyamid MXD 6, Polyamid PDA-T, Polyamid, Polyarylether, Polyaryletherketon, Polyamidimid, Polyarylamid, Polyaminobismaleinimid, Polyarylate, Polybuten-1, Polybutylacrylat, Polybenzimidazol, Polybismaleinimid, Polyoxadiazobenzimidazol, Polybutylenterephthalat, Polycarbonat, Polychlortrifluorethylen, Polyethylen, Polyestercarbonat, Polyaryletherketon, Polyetheretherketon, Polyetherimid, Polyetherketon, Polyethylenoxid, Polyarylethersulfon, Polyethylenterephthalat, Polyimid, Polyisobutylen, Polyisocyanurat, Polyimidsulfon, Polymethacrylimid, Polymethacrylat, Poly-4-methylpenten-1, Polyacetal, Polypropylen, Polyphenylenoxid, Polypropylenoxid, Polyphenylensulfid, Polyphenylensulfon, Polystyrol, Polysulfon, Polytetrafluroethylen, Polyurethan, Polyvinylacetat, Polyvinylalkohol, Polyvinylbutyral, Polyvinylchlorid, Polyvinylidenchlorid, Polyvinylidenfluorid, Polyvinylfluorid, Polyvinylmethylether, Polyvinylpyrrolidon, Styrol-Butadien-Copolymer, Styrol-Isopren-Copolymer, Styrol-Maleinsäureanhydrid-Copolymer, Styrol-Maleinsäureanhydrid-Butadien-Copolymer, Styrol-Methylmethacrylat-Copolymer, Styrol-Methylstyrol-Copolymer, Styrol-Acrylnitril-Copolymer, Vinylchlorid-Ethylen-Copolymer, Vinylchlorid-Methacrylat-Copolymer, Vinylchlorid-Maleinsäureanhydrid-Copolymer, Vinylchlorid-Maleinimid-Copolymer, Vinylchlorid-Methylmethacrylat-Copolymer, Vinylchlorid-Octylacrylat-Copolymer, Vinylchlorid-Vinylacetat-Copolymer, Vinylchlorid-Vinylidenchlorid-Copolymer und Vinylchlorid-Vinylidenchlorid-Acrylnitril-Copolymer.

Weiterhin ist auch die Verwendung von folgenden Kautschuken besonders vorteilhaft: natürlich vorkommendes Polyisopren, insbesondere cis-1,4-Polyisopren (Naturkautschuk; NR) und trans-1,4-Polyisopren (Guttapercha), vor allem Naturkautschuk; Nitrilkautschuk (Copolymer von Butadien und Acrylnitril; Poly(acrylnitril-co-1,3-butadien; NBR; so genannter Buna N-Kautschuk); Butadienkautschuk (Polybutadien; BR); Acrylkautschuk (Polyacrylkautschuk; ACM, ABR); Fluorkautschuk (FPM); Styrol-Butadien-Kautschuk (Copolymer von Styrol und Butadien; SBR); Styrol-Isopren-Butadien-Kautschuk (Copolymer von Styrol, Isopren und Butadien; SIBR); Polybutadien; synthetischer Isoprenkautschuk (Polyisopren; IR); Ethylen-Propylen-Kautschuk (Copolymer von Ethylen und Propylen; EPM); Ethylen-Propylen-Dien-Kautschuk (Terpolymer von Ethylen, Propylen und einer Dien-Komponente; EPDM); Butylkautschuk (Copolymer von Isobutylen und Isopren; IIR); Ethylen-Vinylacetat-Kautschuk (Copolymer von Ethylen und Vinylacetat; EVM); Ethylen-Methylacrylat-Kautschuk (Copolymer von Ethylen und Methylacrylat; AEM); Epoxidkautschuk, wie Polychlormethyloxiran (Epichlorhydrinpolymer; CO), Ethylenoxid (Oxiran) - Chlormethyloxiran (Epichlorhydrinpolymer; ECO), Epichlorhydrin - Ethylenoxid - Allylglycidyletherterpolymer (GECO), Epichlorhydrin - Allylglycidylethercopolymer (GCO) und Propylenoxid - Allylglycidylethercopolymer (GPO); Polynorbornen-Kautschuk (Polymer von Bicyclo[2.2.1]hept-2-en (2-Norbornen); PNR); Polyalkenylen (Polymer von Cycloolefinen); Siliconkautschuk (Q), wie Siliconkautschuk nur mit Methylsubstituenten an der Polymerkette (MQ; z. B. Dimethylpolysiloxan), Siliconkautschuk mit Methylvinyl- und Vinylsubstituentengruppen an der Polymerkette (VMQ), Siliconkautschuk mit Phenyl- und Methylsubstituenten an der Polymerkette (PMQ), Siliconkautschuk mit Fluor- und Methylgruppen an der Polymerkette (FMQ), Siliconkautschuk mit Fluor-, Methyl- und Vinylsubstituenten an der Polymerkette (FVMQ); Polyurethankautschuk; Thiokolkautschuk; Halogenbutylkautschuk, wie Brombutylkautschuk (BIIR) und Chlorbutylkautschuk (CIIR); Chlorpolyethylen (CM); Chlorsulfonylpolyethylen (CSM); hydrierter Nitrilkautschuk (HNBR); und Polyphosphazen.

Besonders bevorzugte Nitrilkautschuke schließen statistische Terpolymere von Acrylnitril, Butadien und einer Carboxylsäure, wie Methacrylsäure, ein. In diesem Zusammenhang umfasst der Nitrilkautschuk vorzugsweise, bezogen auf das Gesamtgewicht des Polymers, die folgenden Hauptkomponenten: 15,0 Gew.-% bis 42,0 Gew.-% Acrylnitrilpolymer; 1,0 Gew.-% bis 10,0 Gew.-% Carbonsäure und der Rest ist überwiegend Butadien (z. B. 38,0 Gew.-% bis 75,0 Gew.-%). Typischerweise ist die Zusammensetzung: 20,0 Gew.-% bis 40,0 Gew.-% Acrylnitrilpolymer, 3,0 Gew.-% bis 8,0 Gew.-% Carbonsäure und 40,0 Gew.-% bis 65,0 Gew.-% oder 67,0 Gew.-% sind Butadien. Besonders bevorzugte Nitrilkautschuke schließen ein Terpolymer von Acrylonitril, Butadien und einer Carbonsäure ein, bei welchem der Acrylnitrilgehalt kleiner als 35,0 Gew.-% ist und der Carbonsäuregehalt kleiner als 10,0 Gew.-% ist, wobei der Butadiengehalt dem verbleibenden Rest entspricht. Noch mehr bevorzugte Nitrilkautschuke können folgende Mengen umfassen: 20,0 Gew.-% bis 30,0 Gew.-% Acrylnitrilpolymer, 4,0 Gew.-% bis 6,0 Gew.-% Carbonsäure und der Rest ist überwiegend Butadien.

Der Einsatz von Stickstoff-haltigen Polymeren, insbesondere von Polyamiden, ist im Rahmen der vorliegenden Erfindung besonders günstig. Besonders bevorzugt werden Polyamid 11, Polyamid 12, Polyamid 46, Polyamid 6, Polyamid 6-3-T, Polyamid 6-Terephthalsäure-Copolymer, Polyamid 66, Polyamid 69, Polyamid 610, Polyamid 612, Polyamid 6I, Polyamid MXD 6 und/oder Polyamid PDA-T, insbesondere Polyamid 12.

Darüber hinaus sind auch ultrahochmolekulare Polyethylene (UHMWPE) für die Zwecke der vorliegenden Erfindung besonders vorteilhaft, insbesondere solche, die eine mittleren Molmasse größer 1000 kg/mol, bevorzugt größer 2000 kg/mol, besonders bevorzugt größer 3000 kg/mol, insbesondere größer 5000 kg/mol aufweisen. Dabei ist das mittlere Molekulargewicht günstigerweise höchstens 10000 kg/mol. Die Dichte besonders geeigneter ultrahochmolekularer Polyethylene liegt im Bereich von 0,94-0,99 g/cm³. Die Kristallinität besonders geeigneter ultrahochmolekularer Polyethylene liegt im Bereich von 50% bis 90%. Die Zugfestigkeit besonders geeigneter ultrahochmolekularer Polyethylene liegt im Bereich von 30N/mm² bis 50N/mm². Das Zug-E-Modul besonders geeigneter ultrahochmolekularer Polyethylene liegt im Bereich von 800 N/mm² bis 2700N/mm². Der Schmelzbereich besonders geeigneter ultrahochmolekularer Polyethylene liegt im Bereich von 135°C bis 155°C.

Weiterhin ist auch die Verwendung von resorbierbaren Polymeren besonders zweckmäßig. Unter dem Begriff "Resorption" (lat. resorbere = "aufsaugen") versteht man die Stoffaufnahme in biologischen Systemen, insbesondere in den menschlichen Organismus. Vorliegend von Interesse sind insbesondere solche Materialien, die für die Herstellung von resorbierbaren Implantaten verwendet werden können.

Erfindungsgemäß besonders bevorzugte, resorbierbare Polymere umfassen Wiederholungseinheiten der Milchsäure, der Hydroxybuttersäure und/oder der Glycolsäure, bevorzugt der Milchsäure und/oder der Glycolsäure, insbesondere der Milchsäure. Polymilchsäuren werden dabei besonders bevorzugt.

Unter "Polymilchsäure" (Polylactiden) werden hier Polymere verstanden, die aus Milchsäureeinheiten aufgebaut sind. Solche Polymilchsäuren werden üblicherweise durch Kondensation von Milchsäuren hergestellt, werden aber auch bei der ringöffnenden Polymerisation von Lactiden unter geeigneten Bedingungen erhalten.

Erfindungsgemäß besonders geeignete, resorbierbare Polymere schließen Poly(glycolid-co-L-lactid), Poly(L-lactid), Poly(L-lactid-co-ε-caprolacton), Poly(L-lactid-co-glycolid), Poly(L-lactid-co-D,L-lactid), Poly(D,L-lactid-co-glycolid) sowie Poly(dioxanon), ein, wobei Milchsäurepolymere, insbesondere Poly-D-, Poly-L- oder Poly-D,L-milchsäuren, vor allem Poly-L-milchsäuren (PLLA) und Poly-D,L-milchsäuren, erfindungsgemäß ganz besonders bevorzugt werden, wobei insbesondere die Verwendung von Poly-L-milchsäuren (PLLA) ganz besonders vorteilhaft ist.

Erfindungsgemäß weist Poly-L-milchsäure (PLLA) vorzugsweise die folgende Struktur auf 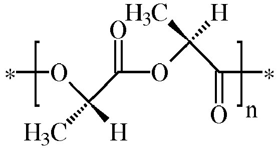 wobei n eine ganze Zahl, vorzugsweise größer 10, ist.

Poly-D,L-milchsäure weist vorzugsweise die folgende Struktur auf 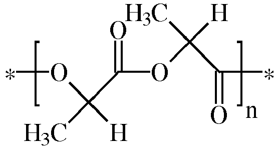 wobei n eine ganze Zahl, vorzugsweise größer 10, ist.

Für die Zwecke der vorliegenden Erfindung geeignete Milchsäurepolymere sind beispielsweise von der Firma Evonik Nutrition & Care GmbH unter den Handelsnamen Resomer^{®} GL 903, Resomer^{®} L 206 S, Resomer^{®} L 207 S, Resomer^{®} R 208 G, Resomer^{®} L 209 S, Resomer^{®} L 210, Resomer^{®} L 210 S, Resomer^{®} LC 703 S, Resomer^{®} LG 824 S, Resomer^{®} LG 855 S, Resomer^{®} LG 857 S, Resomer^{®} LR 704 S, Resomer^{®} LR 706 S, Resomer^{®} LR 708, Resomer^{®} LR 927 S, Resomer^{®} RG 509 S und Resomer^{®} X 206 S kommerziell erhältlich.

Für die Zwecke der vorliegenden Erfindung besonders vorteilhafte, resorbierbare Polymere, bevorzugt handelt es sich hierbei um resorbierbare Polyester, vorzugsweise um Milchsäurepolymere, besonders bevorzugt um Poly-D-, Poly-L- oder Poly-D,L-milchsäuren, insbesondere um Poly-L-milchsäuren, haben ein Zahlenmittel des Molekulargewichts (Mn), vorzugsweise bestimmt durch Gelpermeationschromatographie gegen engverteilte Polystyrol-Standards oder durch Endgruppentitration, größer 500 g/mol, bevorzugt größer 1.000 g/mol, besonders bevorzugt größer 5.000 g/mol, zweckmäßigerweise größer 10.000 g/mol, insbesondere größer 25.000 g/mol. Andererseits ist das Zahlenmittel bevorzugter resorbierbarer Polymere kleiner 1.000.000 g/mol, zweckmäßigerweise kleiner 500.000 g/mol, günstigerweise kleiner 100.000 g/mol, insbesondere höchstens 50.000 g/mol. Ein Zahlenmittel des Molekulargewichts im Bereich von 500 g/mol bis 50.000 g/mol hat sich im Rahmen der vorliegenden Erfindung ganz besonders bewährt.

Das Gewichtsmittel des Molekulargewichts (Mw) bevorzugter resorbierbarer Polymere, vorzugsweise handelt es sich hierbei um resorbierbare Polyester, günstigerweise um Milchsäurepolymere, besonders bevorzugt um Poly-D-, Poly-L- oder Poly-D,L-milchsäuren, insbesondere um Poly-L-milchsäuren, vorzugsweise bestimmt durch Gelpermeationschromatographie gegen engverteilte Polystyrol-Standards, liegt vorzugsweise im Bereich von 750 g/mol bis 5.000.000 g/mol, bevorzugt im Bereich von 750 g/mol bis 1.000.000 g/mol, besonders bevorzugt im Bereich von 750 g/mol bis 500.000 g/mol, insbesondere im Bereich von 750 g/mol bis 250.000 g/mol, und die Polydispersität dieser Polymere ist günstigerweise im Bereich von 1,5 bis 5.

Die inhärente Viskosität besonders geeigneter, resorbierbarer Polymere, bevorzugt handelt es sich hierbei um Milchsäurepolymere, besonders bevorzugt um Poly-D-, Poly-L- oder Poly-D,L-milchsäuren, insbesondere um Poly-L-milchsäuren, gemessen in Chloroform bei 25°C, 0,1 % Polymerkonzentration, liegt im Bereich von 0,3 dl/g bis 8,0 dl/g, bevorzugt im Bereich von 0,5 dl/g bis 7,0 dl/g, besonders bevorzugt im Bereich von 0,8 dl/g bis 2,0 dl/g, insbesondere im Bereich von 0,8 dl/g bis 1,2 dl/g.

Weiterhin ist die inhärente Viskosität besonders geeigneter, resorbierbarer Polymere, bevorzugt handelt es sich hierbei um Milchsäurepolymere, besonders bevorzugt um Poly-D-, Poly-L- oder Poly-D,L-milchsäuren, insbesondere um Poly-L-milchsäuren, gemessen in Hexafluor-2-propanol bei 30°C, 0,1 % Polymerkonzentration, im Bereich von 1,0 dl/g bis 2,6 dl/g, insbesondere im Bereich von 1,3 dl/g bis 2,3 dl/g.

Im Rahmen der vorliegenden Erfindung sind darüber hinaus Polymere, günstigerweise thermoplastische Polymere, bevorzugt Milchsäurepolymere, besonders bevorzugt Poly-D-, Poly-L- oder Poly-D,L-milchsäuren, insbesondere Poly-L-milchsäuren, mit einer Glasübergangstemperatur größer 20°C, günstigerweise größer 25°C, bevorzugt größer 30°C, besonders bevorzugt größer 35°C, insbesondere größer 40°C, äußerst vorteilhaft. Im Rahmen einer ganz besonders bevorzugten Ausführungsform der vorliegenden Erfindung liegt die Glasübergangstemperatur des Polymers im Bereich von 35°C bis 70°C, günstigerweise im Bereich von 55°C bis 65°C, insbesondere im Bereich von 60°C bis 65°C.

Weiterhin sind Polymere, günstigerweise thermoplastische Polymere, bevorzugt Milchsäurepolymere, besonders bevorzugt Poly-D-, Poly-L- oder Poly-D,L-milchsäuren, insbesondere Poly-L-milchsäuren, besonders geeignet, die eine Schmelztemperatur größer 50°C, günstigerweise von mindestens 60°C, bevorzugt von größer 150°C, besonders bevorzugt im Bereich von 130°C bis 210°C, insbesondere im Bereich von 175°C bis 195°C, aufweisen.

Dabei wird die Glastemperatur und die Schmelztemperatur des Polymers vorzugsweise mittels Dynamische Differenzkalorimetrie (Differential Scanning Calorimetry; kurz DSC) ermittelt. Ganz besonders bewährt hat sich in diesem Zusammenhang die folgende Vorgehensweise:
Durchführung der DSC-Messung unter Stickstoff auf einem Mettler-Toledo DSC 30S. Die Eichung erfolgt vorzugsweise mit Indium. Die Messungen werden vorzugsweise unter trockenem, Sauerstoff-freien Stickstoff (Strömungsgeschwindigkeit: vorzugsweise 40 ml/min) durchgeführt. Das Probengewicht wird vorzugsweise zwischen 15 mg und 20 mg gewählt. Die Proben werden zunächst von 0°C auf vorzugsweise eine Temperatur oberhalb der Schmelztemperatur des zu untersuchenden Polymers erwärmt, dann auf 0°C abgekühlt und ein zweites Mal von 0°C auf die genannte Temperatur mit einer Heizrate von 10°C/min erwärmt.

Ganz besonders bevorzugt als thermoplastische Polymere werden Polyamide, UHMWPE sowie resorbierbare Polymere, vor allem resorbierbare Polyester, wie Polybuttersäure, Polyglykolsäure (PGA), Milchsäurepolymere (PLA) und Milchsäurecopolymere, wobei sich Milchsäurepolymere und Milchsäurecopolymere, insbesondere Poly-L-lactid, Poly-D,L-lactid, Copolymere von D,L-PLA und PGA, erfindungsgemäß ganz besonders bewährt haben.

Für die Ziele der vorliegenden Erfindung sind insbesondere die folgenden Polymere ganz besonders geeignet:
1) Poly-L-lactid (PLLA), bevorzugt mit einer inhärenten Viskosität im Bereich von 0,5 dl/g bis 2,5 dl/g, günstigerweise im Bereich von 0,8 dl/g bis 2,0 dl/g, insbesondere im Bereich von 0,8 dl/g bis 1,2 dl/g (jeweils gemessen 0,1% in Chloroform bei 25°C), bevorzugt mit einer Glasübergangstemperatur im Bereich von 60°C bis 65°C, weiterhin bevorzugt mit einer Schmelztemperatur im Bereich von 180°C bis 185°C, darüber hinaus bevorzugt Ester-terminiert;
2) Poly(D,L-lactid), bevorzugt mit einer inhärenten Viskosität im Bereich von 1,0 dl/g bis 3,0 dl/g, günstigerweise im Bereich von 1,5 dl/g bis 2,5 dl/g, insbesondere im Bereich von1,8-2,2 dl/g (jeweils gemessen 0,1% in Chloroform bei 25°C), bevorzugt mit einer Glasübergangstemperatur im Bereich von 55°C bis 60°C,
wobei die besten Ergebnisse unter Verwendung eines Poly-L-lactids erzielt werden, welches bevorzugt eine inhärenten Viskosität im Bereich von 0,5 dl/g bis 2,5 dl/g, günstigerweise im Bereich von 0,8 dl/g bis 2,0 dl/g, insbesondere im Bereich von 0,8 dl/g bis 1,2 dl/g aufweist (jeweils gemessen 0,1% in Chloroform bei 25°C), bevorzugt eine Glasübergangstemperatur im Bereich von 60°C bis 65°C hat, weiterhin bevorzugt eine Schmelztemperatur im Bereich von 180°C bis 185°C aufweist und darüber hinaus bevorzugt Ester-terminiert ist.

Im Rahmen der vorliegenden Erfindung umfassen die für die Herstellung des erfindungsgemäßen Verbundpulvers einsetzbaren kleinen Teilchen (zweites Material) kugelförmige gefällte Calciumcarbonat-Teilchen. Im Unterschied zu anderen bekannten Formen des Standes der Technik sind die Calciumcarbonat-Teilchen also nicht z. B. aus Nadeln, Rhomboedern bzw. Skalenoeder (gefälltes Calciumcarbonat; PCC) oder aus unregelmäßig geformten Teilchen (gemahlenes Calciumcarbonat; GCC), sondern aus kugelförmigen gefällten Teilchen aufgebaut, die vorzugsweise überwiegend einzelteilig vorliegen. Es werden jedoch kleinere Abweichungen von der perfekten Kugelform akzeptiert, solange die Eigenschaften der Teilchen, insbesondere ihre Dispergierbarkeit, nicht grundlegend verändert werden. So kann die Oberfläche der Teilchen gelegentliche Fehlstellen oder zusätzliche Ablagerungen aufweisen.

Der mittlere Durchmesser der kugelförmigen Calciumcarbonat-Teilchen ist im Bereich von 0,05 µm bis 50,0 µm, insbesondere im Bereich von 2,5 µm bis 30,0 µm. Dabei ist der mittlere Teilchendurchmesser zweckmäßigerweise größer 2,5 µm, günstigerweise größer 3,0 µm, vorzugsweise größer 4,0 µm, zweckmäßigerweise größer 5,0 µm, zweckmäßigerweise größer 6,0 µm, bevorzugt größer 7,0 µm, besonders bevorzugt größer 8,0 µm, noch mehr bevorzugt größer 9,0 µm, ganz besonders bevorzugt größer 10,0 µm, noch mehr bevorzugt größer 11,0 µm, vor allem größer 12,0 µm, insbesondere größer 13,0 µm. Weiterhin ist der mittlere Teilchendurchmesser zweckmäßigerweise kleiner 30,0 µm, günstigerweise kleiner 20,0 µm, bevorzugt kleiner 18,0 µm, besonders bevorzugt kleiner 16,0 µm, insbesondere kleiner 14,0 µm.

Im Rahmen der vorliegenden Erfindung wird der mittlere Durchmesser der Calciumcarbonat-Teilchen zweckmäßigerweise durch Auswertung von Rasterelektronenmikroskop-Aufnahmen (REM-Aufnahmen) ermittelt, wobei vorzugsweise nur Teilchen mit einer Größe von mindestens 0,01 µm berücksichtigt werden und ein Zahlenmittel über vorzugsweise mindestens 20, besonders bevorzugt mindestens 40 Teilchen gebildet wird. Weiterhin haben sich auch Sedimentationsanalyseverfahren besonders bewährt, wobei in diesem Zusammenhang die Verwendung eines Sedigraphs 5100 (Micromeritics GmbH) besonders vorteilhaft ist.

Die Größenverteilung der Calciumcarbonat-Teilchen ist zweckmäßigerweise vergleichsweise eng und vorzugsweise derart, dass mindestens 90,0 Gew.-% aller Calciumcarbonat-Teilchen, einen Teilchendurchmesser im Bereich von mittlerer Teilchendurchmesser -30 % bis mittlerer Teilchendurchmesser +30 % aufweisen. Der Formfaktor der Calciumcarbonat-Teilchen, vorliegend definiert als der Quotient aus minimalem Teilchendurchmesser und maximalem Teilchendurchmesser, ist zweckmäßigerweise für mindestens 90 %, günstigerweise für mindestens 95 % aller Teilchen, größer 0,90, besonders bevorzugt größer 0,95. In diesem Zusammenhang werden vorzugsweise nur Teilchen mit einer Teilchengröße im Bereich von 0,1 µm bis 50,0 µm, insbesondere im Bereich von 0,1 µm bis 30,0 µm, berücksichtigt.

Die Calciumcarbonat-Teilchen zeichnen sich günstigerweise weiterhin durch einen vergleichsweise geringen Wassergehalt aus. Sie weisen, bezogen auf ihr Gesamtgewicht, zweckmäßigerweise einen Wassergehalt (Restfeuchte bei 200°C) von höchstens 5,0 Gew.-%, vorzugsweise von höchstens 2,5 Gew.-%, bevorzugt von höchstens 1,0 Gew.-%, besonders bevorzugt von höchstens 0,5 Gew.-%, noch mehr bevorzugt kleiner 0,4 Gew.-%, zweckmäßigerweise kleiner 0,3 Gew.-%, günstigerweise kleiner 0,2 Gew.-%, insbesondere im Bereich von >0,1 Gew.-% bis <0,2 Gew.-%, auf.

Im Rahmen der vorliegenden Erfindung wird der Wassergehalt der Calciumsalz-Teilchen, insbesondere der Calciumcarbonat-Teilchen, vorzugsweise mittels Thermogravimetrie oder mittels eines Infrarotschnelltrockners, z. B. MA35 oder MA45 der Firma Sartorius oder Halogen-Feuchtigkeitsmessgerät HB43 der Firma Mettler, ermittelt, wobei die Messung vorzugsweise unter Stickstoff (Stickstoff-Durchflußmenge bevorzugt 20 ml/min) und zweckmäßigerweise über den Temperaturbereich von 40°C oder niedriger bis 250°C oder höher durchgeführt wird. Weiterhin erfolgt die Messung vorzugsweise bei einer Heizrate von 10°C/min.

Die spezifische Oberfläche der Calciumcarbonat-Teilchen ist vorzugsweise kleiner 3,0 m²/g, bevorzugt kleiner 2,0 m²/g, insbesondere kleiner 1,5 m²/g. Weiterhin ist die spezifische Oberfläche günstigerweise größer 0,25 m²/g, vorzugsweise größer 0,5 m²/g, insbesondere größer 0,75 m²/g.

Im Rahmen einer besonders bevorzugten Variante der vorliegenden Erfindung sind die gefällten Calciumcarbonat-Teilchen kugelförmig und im Wesentlichen amorph. Der Begriff "amorph" bezeichnet an dieser Stelle solche Calciumcarbonat-Modifikationen, bei welchen die Atome zumindest teilweise keine geordneten Strukturen, sondern ein unregelmäßiges Muster ausbilden und daher nur über eine Nahordnung, nicht aber über eine Fernordnung verfügen. Hiervon zu unterscheiden sind kristalline Modifikationen des Calciumcarbonats, wie z. B. Calcit, Vaterit und Aragonit, bei welchen die Atome sowohl eine Nah- als auch eine Fernordnung aufweisen.

Im Rahmen dieser bevorzugten Variante der vorliegenden Erfindung wird die Gegenwart von kristallinen Bestandteilen jedoch nicht kategorisch ausgeschlossen. Vorzugsweise ist der Anteil von kristallinem Calciumcarbonat jedoch kleiner 50 Gew.-%, besonders bevorzugt kleiner 30 Gew.-%, ganz besonders bevorzugt kleiner 15 Gew.-%, insbesondere kleiner 10 Gew.-%. Im Rahmen einer besonders bevorzugten Variante der vorliegenden Erfindung ist der Anteil von kristallinem Calciumcarbonat kleiner 8,0 Gew.-%, bevorzugt kleiner 6,0 Gew.-%, zweckmäßigerweise kleiner 4,0 Gew.-%, besonders bevorzugt kleiner 2,0 Gew.-%, ganz besonders bevorzugt kleiner 1,0 Gew.-%, insbesondere kleiner 0,5 Gew.-% , jeweils bezogen auf das Gesamtgewicht des Calciumcarbonats.

Für die Ermittlung der amorphen und der kristallinen Anteile hat sich die Röntgenbeugung mit einem internen Standard, vorzugsweise Quarz, in Verbindung mit einer Rietveld-Verfeinerung ganz besonders bewährt.

Im Rahmen dieser bevorzugten Ausführungsform der vorliegenden Erfindung werden die vorzugsweise amorphen Calciumcarbonat-Teilchen günstigerweise durch mindestens eine Substanz, insbesondere mindestens eine grenzflächenaktive Substanz, stabilisiert, die vorzugsweise auf der Oberfläche der vorzugsweise kugelförmigen Calciumcarbonat-Teilchen angeordnet ist. "Grenzflächenaktive Substanzen" bezeichnen im Sinne der vorliegenden Erfindung zweckmäßigerweise organische Verbindungen, die sich aus ihrer Lösung an Grenzflächen (Wasser/Calciumcarbonat-Teilchen) stark anreichern und dadurch die Oberflächenspannung, vorzugsweise gemessen bei 25°C, herabsetzen. Für weitere Details wird auf die Fachliteratur, insbesondere auf Römpp-Lexikon Chemie / Hrsg. Jürgen Falbe; Manfred Regitz. Bearb. Von Eckard Amelingmeier; Stuttgart, New York; Thieme; Band 2: Cm-G; 10. Auflage (1997); Stichwort: "grenzflächenaktive Stoffe", verwiesen.

Vorzugsweise weist die Substanz, insbesondere die grenzflächenaktive Substanz, eine Molmasse größer 100 g/mol, bevorzugt größer 125 g/mol, insbesondere größer 150 g/mol, auf und genügt der Formel R-Xₙ.

Der Rest R steht dabei für einen mindestens 1, vorzugsweise mindestens 2, bevorzugt mindestens 4, besonders bevorzugt mindestens 6, insbesondere mindestens 8, Kohlenstoffatome umfassenden Rest, vorzugsweise für einen aliphatischen oder cycloaliphatischen Rest, der ggf. weitere Reste X umfassen kann und der ggf. eine oder mehrere Etherverknüpfungen aufweisen kann.

Der Rest X steht für eine Gruppe, die mindestens ein Sauerstoffatom sowie mindestens ein Kohlenstoffatom, Schwefelatom, Phosphoratom und/oder Stickstoffatom, bevorzugt mindestens ein Phosphoratom und/oder mindestens ein Kohlenstoffatom, umfasst. Besonders bevorzugt werden die folgenden Gruppen:
Carbonsäuregruppen -COOH,
Carboxylatgruppen ~COO⁻,
Sulfonsäuregruppen ~SO₃H,
Sulfonatgruppen ~SO₃⁻,
Hydrogensulfatgruppen ~OSO₃H,
Sulfatgruppen ~OSO₃⁻,
Phosphonsäuregruppen ~PO₃H₂,
Phosphonatgruppen ~PO₃H-, ~PO₃²⁻,
Aminogruppen ~NR¹R² sowie
Ammoniumgruppen ~N⁺R¹R²R³,
insbesondere Carbonsäuregruppen, Carboxylatgruppen, Phosphonsäuregruppen und Phosphonatgruppen.

Die Reste R¹, R² und R³ stehen in diesem Zusammenhang unabhängig voneinander für Wasserstoff oder eine Alkylgruppe mit 1 bis 5 Kohlenstoffatomen. Einer der Reste R¹, R² und R³ kann auch ein Rest R sein.

Bevorzugte Gegenionen für die vorstehend genannten Anionen sind Metallkationen, insbesondere Alkalimetallkationen, bevorzugt Na⁺ und K⁺, sowie Ammoniumionen.

Bevorzugte Gegenionen für die vorstehend genannten Kationen sind Hydroxylionen, Hydrogencarbonationen, Carbonationen, Hydrogensulfationen, Sulfationen und Halogenidionen, insbesondere Chlorid- und Bromidionen.

n steht für eine vorzugsweise ganze Zahl im Bereich von 1 bis 20, bevorzugt im Bereich von 1 bis 10, insbesondere im Bereich von 1 bis 5.

Für die Zwecke der vorliegenden Erfindung besonders geeignete Substanzen umfassen Alkylcarbonsäuren, Alkylcarboxylate, Alkylsulfonsäuren, Alkylsulfonate, Alkylsulfate, Alkylethersulfate mit vorzugsweise 1 bis 4 Ethylenglykolethereinheiten, Fettalkoholethoxylate mit vorzugsweise 2 bis 20 Ethylenglykolethereinheiten, Alkylphenolethoxylate, ggf. substituierte Alkylphosphonsäuren, ggf. substituierte Alkylphosphonate, Sorbitanfettsäureester, Alkylpolyglucoside, N-Methylglucamide, Homo- und Copolymere der Acrylsäure sowie deren entsprechenden Salzformen und Blockcopolymere.

Eine erste Gruppe ganz besonders vorteilhafter Substanzen sind ggf. substituierte Alkylphosphonsäuren, insbesondere Amino-tri-(methylenphosphonsäure), 1-Hydroxyethylen-(1,1-diphosphonsäure), Ethylendiamin-tetra-(methylenphosphonsäure), Hexamethylendiamin-tetra-(methylenphosphonsäure), Diethylentriamin-penta-(methylenphosphonsäure), sowie ggf. substituierte Alkylphosphonate, insbesondere der vorstehend genannten Säuren. Diese Verbindungen sind als multifunktionelle Sequestriermittel für Metallionen und Steininhibitoren bekannt.

Weiterhin haben sich auch Homo- und Copolymere, bevorzugt Homopolymere, der Acrylsäure sowie deren entsprechenden Salzformen besonders bewährt, insbesondere solche, die ein Gewichtsmittel des Molekulargewichts im Bereich von 1.000 g/mol - 10.000 g/mol aufweisen.

Ferner ist die Verwendung von Blockcopolymeren, bevorzugt von doppelhydrophilen Blockcopolymeren, insbesondere von Polyethylenoxid oder Polypropylenoxid, besonders günstig.

Der Anteil der vorzugsweise grenzflächenaktiven Substanzen kann prinzipiell frei gewählt und für die jeweilige Anwendung gezielt eingestellt werden. Er liegt jedoch vorzugsweise im Bereich von 0,1 Gew.-% bis 5,0 Gew.-%, insbesondere im Bereich von 0,3 Gew.-% bis 1,0 Gew.-%, bezogen auf den Calciumcarbonat-Gehalt der Teilchen.

Die Herstellung der kugelförmigen Calciumcarbonat-Teilchen erfolgt kann durch Carbonatisierung einer wässrigen Calciumhydroxid (Ca(OH)₂)-Suspension. Hierzu wird zweckmäßigerweise CO₂ oder ein CO₂-haltiges Gasgemisch in eine Calciumhydroxid-Suspension geleitet.

Besonders bewährt hat sich eine Vorgehensweise, bei welcher man
a. eine wässrige Calciumhydroxid-Suspension vorlegt,
b. in die Suspension aus Schritt a. Kohlendioxid oder eine Kohlendioxid-haltige Gasmischung einleitet und
c. die entstehenden Calciumcarbonat-Teilchen abtrennt,
wobei man weiterhin 0,3 Gew.-% bis 0,7 Gew.-%, bevorzugt 0,4 Gew.-% bis 0,6 Gew.-%, insbesondere 0,45 Gew.-% bis 0,55 Gew.-%, mindestens einer Aminotrialkylenphosphonsäure zugibt.

Die Konzentration der Calciumhydroxid -Suspension unterliegt keinen besonderen Beschränkungen. Besonders günstig ist jedoch eine Konzentrationen im Bereich von 1 g CaO/l bis 100 g CaO/l, bevorzugt im Bereich von 10 g CaO/l bis 90 g CaO/l, insbesondere im Bereich von 50 g CaO/l bis 80 g CaO/l.

Als Aminotrialkylenphosphonsäure gibt man vorzugsweise Aminotrimethylenphosphonsäure, Aminotriethylenphosphonsäure, Aminotripropylenphosphonsäure und/oder Aminotributylenphosphonsäure, insbesondere Aminotrimethylenphosphonsäure, zu.

Über die Menge an eingeleitetem CO₂ kann der Umsatz der Reaktion gesteuert werden. Jedoch führt man die Einleitung des Kohlendioxids oder der Kohlendioxid-haltigen Gasmischung vorzugsweise solange durch, bis die Reaktionsmischung einen pH-Wert kleiner 9, bevorzugt kleiner 8, insbesondere kleiner 7,5, aufweist.

Weiterhin leitet man das Kohlendioxid oder die Kohlendioxid-haltige Gasmischung zweckmäßigerweise mit einer Gasdurchflußmenge im Bereich von 0,02 l CO₂ / (h*g Ca(OH)₂) bis 2,0 l CO₂ / (h*g Ca(OH)₂), bevorzugt im Bereich von 0,04 l CO₂ / (h*g Ca(OH)₂) bis 1,0 l CO₂ / (h*g Ca(OH)₂), besonders bevorzugt im Bereich von 0,08 l CO₂ / (h*g Ca(OH)₂) bis 0,4 l CO₂ / (h*g Ca(OH)₂), insbesondere im Bereich von 0,12 l CO₂ / (h*g Ca(OH)₂) bis 0,2 l CO₂ / (h*g Ca(OH)₂), in die Calciumhydroxid-Suspension ein.

Im Übrigen erfolgt die Umsetzung der Calciumhydroxid -Suspension mit dem Kohlendioxid oder der Kohlendioxid-haltigen Gasmischung vorzugsweise bei einer Temperatur kleiner 25°C, bevorzugt kleiner 20°C, insbesondere kleiner 15°C. Andererseits ist die Reaktionstemperatur vorzugsweise größer 0°C, bevorzugt größer 5°C, insbesondere größer 7°C.

Die Zugabe der mindestens einen Aminotrialkylenphosphonsäure erfolgt zweckmäßigerweise im Verlauf der Reaktion, bevorzugt nach einem abrupten Abfall des Leitwerts der Reaktionsmischung. Zweckmäßigerweise wird die mindestens eine Aminotrialkylenphosphonsäure zugegeben, sobald die Leitfähigkeit der Reaktionsmischung um mehr als 0,5 mS/cm/min absinkt. Die Abnahme des Leitwerts der Reaktionsmischung beträgt dabei vorzugsweise mindestens 0,25 mS/cm innerhalb von 30 Sekunden, insbesondere mindestens 0,5 mS/cm innerhalb von 60 Sekunden. Im Rahmen einer besonders bevorzugten Ausführungsform der vorliegenden Erfindung erfolgt die Zugabe der mindestens einen Aminotrialkylenphosphonsäure am Ende der Fällung des basischen Calciumcarbonats (BCC; 2CaCO₃*Ca(OH)₂*nH₂O).

Die Calciumcarbonat-Teilchen fallen unter den oben genannten Bedingungen aus der Reaktionsmischung aus und können auf an sich bekannte Weise abgetrennt und getrocknet werden.

Im Rahmen einer bevorzugten Ausführungsform der vorliegenden Erfindung enthält das erfindungsgemäße Verbundpulver neben Calciumcarbonat noch andere Calciumsalze, bevorzugt Calciumphosphate, insbesondere Ca₃(PO₄)₂, CaHPO₄, Ca(H₂PO₄)₂ und/oder Cas(PO₄)₃(OH). Das Gewichtsverhältnis von Calciumcarbonat zu Calciumphosphat liegt dabei vorzugsweise im Bereich von 99:1 bis 1:99, insbesondere im Bereich von 50:50 bis 99:1.

Im Rahmen einer bevorzugten Ausführungsform der vorliegenden Erfindung umfassen die kleinen Teilchen inhibierende Calciumcarbonat-Teilchen. "Inhibierende Calciumcarbonat-Teilchen" bezeichnen in diesem Zusammenhang Calciumcarbonat-Teilchen, die als Additiv in Polymeren den säurekatalysierten Abbau des Polymers im Vergleich mit demselben Polymer ohne Additiv verlangsamen, im besten Fall vollständig unterdrücken.

Zweckmäßigerweise sind die kleinen Teilchen durch ein Verfahren erhältlich, bei welchem man Calciumcarbonat-Teilchen mit einer Zusammensetzung beschichtet, die, bezogen auf ihr Gesamtgewicht, mindestens 0,1 Gew.-% mindestens einer schwachen Säure umfasst.

Im Rahmen einer besonders bevorzugten Ausführungsform der vorliegenden Erfindung ist das inhibierende Calciumcarbonat durch ein Verfahren erhältlich, bei welchem man Calciumcarbonat-Teilchen mit einer Zusammensetzung beschichtet, die, jeweils bezogen auf ihr Gesamtgewicht, eine Mischung von mindestens 0,1 Gew.-% mindestens eines Calcium-Komplexbildners und/oder mindestens einer konjugierten Base, die ein Alkalimetall- oder Calciumsalz einer schwachen Säure ist, gemeinsam mit mindestens 0,1 Gew.-% mindestens einer schwachen Säure umfasst.

Die Anionen des Calcium-Komplexbildners und der konjugierten Base können im Rahmen dieser Ausführungsform gleich sein, obgleich dies keine zwingende Voraussetzung ist.

Als Calcium-Komplexbildner haben sich Natriumphosphate, d.h. Natriumsalze von Phosphorsäuren, insbesondere Natriumsalze von Orthophosphorsäure, Metaphosphorsäure und Polyphosphorsäure, als ganz besonders vorteilhaft erwiesen. Bevorzugte Natriumphosphate umfassen Natriumorthophosphate, wie primäres Natriumdihydrogenphosphat NaH₂PO₄, sekundäres Natriumdihydrogenphosphat Na₂HPO₄ und tertiäres Trinatriumphosphat Na₃PO₄; Natriumisopolyphosphate, wie Tetranatriumdiphosphat (Natriumpyrophosphat) Na₄P₂O₇, Pentanatriumtriphosphat (Natriumtripolyphosphat) Na₅P₃O₁₀; sowie höhermolekulare Natriumphosphate, wie Natriummetaphosphate und Natriumpolyphosphate, wie Schmelz- oder Glühphosphate, Grahamsches Salz (ungefähre Zusammensetzung Na₂O*P₂O₅, gelegentlich auch als Natriumhexametaphosphat bezeichnet), Kurrolsches Salz und Maddrellsches Salz. Ganz besonders bevorzugt wird erfindungsgemäß Natriumhexametaphosphat verwendet. Die Verwendung von den vorstehend genannten Phosphaten ist insbesondere in einem Verbundpulver für Implantate besonders vorteilhaft, da in diesem Fall die Phosphate den Knochenbau zusätzlich fördern.

Weitere geeignete Calcium-Komplexbildner schließen gemeinsame mehrzähnige, chelatbildende Liganden ein, insbesondere Ethylendiamintetraessigsäure (EDTA), Triethylentetramin, Diethylentriamin, o-Phenanthrolin, Oxalsäure und Mischungen davon.

Für die Zwecke der vorliegenden Erfindung besonders geeignete schwache Säuren weisen einen pKs-Wert, gemessen bei 25°C, größer 1,0, bevorzugt größer 1,5, insbesondere größer 2,0, auf. Gleichzeitig ist der pKs-Wert geeigneter schwacher Säuren, gemessen bei 25°C, vorzugsweise kleiner 20,0, bevorzugt kleiner 10,0, besonders bevorzugt kleiner 5,0, zweckmäßigerweise kleiner 4,0, insbesondere kleiner 3,0. Erfindungsgemäß ganz besonders geeignete schwache Säuren umfassen Phosphorsäure, Metaphosphorsäure, Hexametaphosphorsäure, Zitronensäure, Borsäure, schweflige Säure, Essigsäure und Mischungen davon. Phosphorsäure wird als schwache Säure ganz besonders bevorzugt eingesetzt.

Erfindungsgemäß bevorzugte konjugierte Basen schließen insbesondere Natrium- oder Calciumsalze der vorstehend genannten schwachen Säuren ein, wobei Natriumhexametaphosphat ganz besonders bevorzugt wird.

Die Herstellung der inhibierenden Calciumcarbonat-Teilchen kann auf an sich bekannte Weise durch Beschichten von Calciumcarbonat-Teilchen mit einer Zusammensetzung erfolgen, die mindestens eine schwache Säure umfasst.

Besonders bevorzugt erfolgt die Herstellung der inhibierenden Calciumcarbonat-Teilchen auf an sich bekannte Weise durch Beschichten von Calciumcarbonat-Teilchen mit einer Zusammensetzung, die mindestens einen Calcium-Komplexbildner und/oder mindestens eine konjugierte Base, die ein Alkalimetall- oder Calciumsalz einer schwachen Säure ist, gemeinsam mit mindestens einer schwachen Säure umfasst. Die gleichzeitige Beschichtung mit mindestens einem Calcium-Komplexbildner und/oder mindestens einer konjugierten Base, die ein Alkalimetall- oder Calciumsalz einer schwachen Säure ist, gemeinsam mit mindestens einer schwachen Säure führt zu besonders bevorzugten Calciumcarbonat-Teilchen.

Zweckmäßigerweise wird eine wässrige Suspension der zu beschichtenden Calciumcarbonat-Teilchen vorgelegt, die, bezogen auf ihr Gesamtgewicht, günstigerweise einen Gehalt von Calciumcarbonat-Teilchen im Bereich von 1,0 Gew.-% bis 80,0 Gew.-%, bevorzugt im Bereich von 5,0 Gew.-% bis 50,0 Gew.-%, insbesondere im Bereich von 10,0 Gew.-% bis 25,0 Gew.-%, aufweist.

Die Beschichtung der Calciumcarbonat-Teilchen erfolgt günstigerweise durch Zugabe der genannten Substanz oder Substanzen in Reinform oder in wässriger Lösung, wobei sich wässrige Lösungen der genannten Komponente oder Komponenten erfindungsgemäß als ganz besonders vorteilhaft erwiesen haben, um eine möglichst homogene Beschichtung der Calciumcarbonat-Teilchen zu erreichen.

Weiterhin ist es im Rahmen der vorliegenden Erfindung besonders günstig, den Calcium-Komplexbildner und/oder die konjugierte Base, die ein Alkalimetall- oder Calciumsalz einer schwachen Säure ist, vor der schwachen Säure zuzugeben.

Der Calcium-Komplexbildner oder die konjugierte Base wird vorzugsweise in einer Menge im Bereich von 0,1 Gew.-Teile bis 25,0 Gew.-Teile, bevorzugt im Bereich von 0,5 Gew.-Teile bis 10,0 Gew.-Teile, insbesondere im Bereich von 1,0 Gew.-Teile bis 5,0 Gew.-Teile, jeweils bezogen auf 100 Gew.-Teile der zu beschichtenden Calciumcarbonat-Teilchen, verwendet. Dabei wird die Menge des Calcium-Komplexbildners oder der konjugierten Base zweckmäßigerweise derart gewählt, dass eine vollständige Beschichtung der Oberfläche der Calciumcarbonat-Teilchen mit dem Calcium-Komplexbildner der konjugierten Base erreicht wird.

Die schwache Säure wird vorzugsweise in einer Menge im Bereich von 0,1 Gew.-Teile bis 30,0 Gew.-Teile, bevorzugt im Bereich von 0,5 Gew.-Teile bis 15,0 Gew.-Teile, besonders bevorzugt im Bereich von 1,0 Gew.-Teile bis 10,0 Gew.-Teile, insbesondere im Bereich von 4,0 Gew.-Teile bis 8,0 Gew.-Teile, jeweils bezogen auf 100 Gew.-Teile der zu beschichtenden Calciumcarbonat-Teilchen, eingesetzt.

Die auf diese Weise erhältlichen inhibierenden Calciumcarbonat-Teilchen sind in einer mäßig sauren Umgebung stabil, wobei diese Fähigkeit auf eine Pufferwirkung durch die schwache Säure, bevorzugt durch den absorbierten oder umgesetzten Calcium-Komplexbildner oder der konjugierten Base auf der Oberfläche der Calciumcarbonat-Teilchen und die schwache Säure zurückgeführt wird, wobei insbesondere die Aufbringung des Calcium-Komplexbildners und/oder der konjugierte Base auf der Oberfläche der Calciumcarbonat-Teilchen wiederum die Löslichkeit der Oberfläche der Calciumcarbonat-Teilchens herabsetzt und somit die Calciumcarbonat-Teilchen stabilisiert, ohne dass die Lehre der vorliegenden Erfindung an diese Theorie gebunden sein soll.

Erfindungsgemäß ist das Verbundpulver durch ein Verfahren erhältlich, bei welchem man große Teilchen mit kleinen Teilchen verbindet, wobei
- die großen Teilchen einen mittleren Teilchendurchmesser im Bereich von 0,1 µm bis 10 mm, bevorzugt im Bereich von 5 µm bis 10 mm, besonders bevorzugt im Bereich von 10 µm bis 10 mm, günstigerweise im Bereich von 20 µm bis 10 mm, vorteilhafterweise im Bereich von 30 µm bis 2,0 mm, insbesondere im Bereich von 60,0 µm bis 500,0 µm, aufweisen,
- der mittlere Teilchendurchmesser der kleinen Teilchen vorzugsweise höchstens 1/5, bevorzugt höchstens 1/10, besonders bevorzugt höchstens 1/20, insbesondere höchstens 1/1000, des mittleren Teilchendurchmessers der großen Teilchen ist.

Dabei sind die kleinen Teilchen auf der Oberfläche der großen Teilchen angeordnet und/oder inhomogen innerhalb der großen Teilchen verteilt. Im Rahmen einer besonders bevorzugten Ausführungsform der vorliegenden Erfindung, insbesondere für resorbierbare Polymere und für UHMWPE, werden allerdings hervorragende Ergebnisse erzielt, wenn die kleinen Teilchen zumindest teilweise auf der Oberfläche der großen Teilchen angeordnet sind und diese vorzugsweise nicht vollständig bedecken. Ganz besonders bevorzugt sind die kleinen Teilchen auf der Oberfläche der großen Teilchen angeordnet und bedecken diese vorzugsweise nicht vollständig.

Eine "inhomogene" Verteilung der kleinen Teilchen oder Fragmente davon innerhalb der großen Teilchen bedeutet hier eine nicht homogene (gleichmäßige) Verteilung der kleinen Teilchen oder Fragmente davon innerhalb der großen Teilchen. Vorzugsweise gibt es innerhalb der Teilchen des Verbundpulvers mindestens einen ersten Bereich, der mindestens zwei, vorzugsweise mindestens drei, bevorzugt mindestens vier, insbesondere mindestens fünf kleine Teilchen oder Fragmente davon umfasst, und mindestens einen anderen Bereich innerhalb der Teilchen des Verbundpulvers, der zwar das gleiche Volumen und die gleiche Form wie der erste Bereich aufweist, aber eine andere Anzahl von kleinen Teilchen umfasst.

Im Rahmen einer bevorzugte Ausführungsform der vorliegenden Erfindung ist das Gewichtsverhältnis Polymer, insbesondere Polyamid, zu gefälltem Calciumcarbonat im Teilcheninneren größer als das Gewichtsverhältnis Polymer, insbesondere Polyamid, zu gefälltem Calciumcarbonat im Außenbereich der Teilchen. Zweckmäßigerweise ist das Gewichtsverhältnis Polymer, insbesondere Polyamid, zu gefälltem Calciumcarbonat im Teilcheninneren größer 50:50, bevorzugt größer 60:40, günstigerweise größer 70:30, besonders bevorzugt größer 80:20, noch mehr bevorzugt größer 90:10, ganz besonders bevorzugt größer 95:5, insbesondere größer 99:1. Weiterhin ist das Gewichtsverhältnis gefälltes Calciumcarbonat zu Polymer, insbesondere Polyamid, im Außenbereich der Teilchen, bevorzugt im Vorzugsaußenbereich der Teilchen, größer 50:50, bevorzugt größer 60:40, günstigerweise größer 70:30, besonders bevorzugt größer 80:20, noch mehr bevorzugt größer 90:10, ganz besonders bevorzugt größer 95:5, insbesondere größer 99:1.

Im Rahmen einer weiteren bevorzugten Ausführungsform der vorliegenden Erfindung sind die kleinen Teilchen auf der Oberfläche der großen Teilchen angeordnet und bedecken die großen Teilchen vorzugsweise nicht vollständig.. Zweckmäßigerweise sind mindestens 0,1 %, bevorzugt mindestens 5,0 %, insbesondere 50,0 %, der Oberfläche der großen Teilchen nicht mit den kugelförmigen Calciumcarbonat-Teilchen beschichtet. Verstärkt wird dieser Effekt vorzugsweise durch die Lücken zwischen einzelnen Calciumcarbonat-Teilchen, die vorzugsweise vorliegen und zur Ausbildung von entsprechenden Mikrokanälen für fluide Substanzen, insbesondere für eine Schmelze des Polymers der großen Teilchen, führen. Diese Struktur ist insbesondere für Anwendungen des Verbundpulvers in Lasersinterverfahren vorteilhaft, da hierdurch ein gleichmäßiges und schnelles Aufschmelzen des im Verbundpulver enthaltenden Polymers, bevorzugt des thermoplastischen Polymers, besonders bevorzugt des resorbierbaren Polymers, insbesondere des Milchsäurepolymers, sichergestellt wird.

Im Rahmen der vorliegenden Erfindung ist das erfindungsgemäße Verbundpulver durch eine spezielle Teilchengrößenverteilung gekennzeichnet. Die Teilchen des Verbundpulvers weisen eine mittlere Teilchengröße dso im Bereich von 10 µm bis kleiner 200 µm, bevorzugt im Bereich von 20 µm bis kleiner 200 µm, besonders bevorzugt im Bereich von 20 µm bis kleiner 150 µm, günstigerweise im Bereich von 20 µm bis kleiner 100 µm, insbesondere im Bereich von 35 µm bis kleiner 70 µm, auf.

Weiterhin ist Feinkornanteil des Verbundpulvers vorzugsweise kleiner 50,0 Vol.-%, bevorzugt kleiner 45,0 Vol.-%, besonders bevorzugt kleiner 40,0 Vol.-%, noch mehr bevorzugt kleiner 20,0 Vol.-%, günstigerweise kleiner 15,0 Vol.-%, zweckmäßigerweise kleiner 10,0 Vol.-%, insbesondere kleiner 5,0 Vol.-%. Dabei bezeichnet der Feinkornanteil erfindungsgemäß den Anteil der kleinsten Partikelpopulation bei einer bi- oder multimodalen Korngrößenverteilung bezogen auf die Gesamtmenge bei der Summenverteilungskurve. Bei einer unimodalen (monodispersen) Korngrößenverteilung wird der Feinkornanteil erfindungsgemäß als 0,0 Vol.-% definiert. Berücksichtigt werden in diesem Zusammenhang alle im Produkt vorliegenden Partikel inklusive nicht verbundenes Ausgangsmaterial, insbesondere kleine Teilchen im Sinne der Erfindung sowie Bruchstücke oder Fragmente der großen und/oder der kleinen Teilchen im Sinne der Erfindung.

Für Verbundpulver mit einer mittleren Teilchengröße dso im Bereich von größer 40 µm bis kleiner 200 µm ist der Feinkornanteil vorzugsweise derart, dass der Anteil von Teilchen im Produkt mit einer Teilchengröße kleiner 20 µm vorzugsweise kleiner 50,0 Vol.-%, bevorzugt kleiner 45,0 Vol.-%, besonders bevorzugt kleiner 40,0 Vol.-%, noch mehr bevorzugt kleiner 20,0 Vol.-%, günstigerweise kleiner 15,0 Vol.-%, zweckmäßigerweise kleiner 10,0 Vol.-%, insbesondere kleiner 5,0 Vol.-%, ist, wobei "Teilchen" in diesem Zusammenhang insbesondere Teilchen des Verbundpulvers im Sinne der Erfindung, kleine Teilchen im Sinne der Erfindung sowie Bruchstücke oder Fragmente der großen und/oder der kleinen Teilchen im Sinne der Erfindung umfassen, sofern sie die genannte Teilchengröße aufweisen.

Für Verbundpulver mit einer mittleren Teilchengröße dso im Bereich von 10 µm bis 40 µm ist der Feinkornanteil vorzugsweise derart, dass der Anteil von Teilchen im Produkt mit einer Teilchengröße kleiner 5 µm vorzugsweise kleiner 50,0 Vol.-%, bevorzugt kleiner 45,0 Vol.-%, besonders bevorzugt kleiner 40,0 Vol.-%, noch mehr bevorzugt kleiner 20,0 Vol.-%, günstigerweise kleiner 15,0 Vol.-%, zweckmäßigerweise kleiner 10,0 Vol.-%, insbesondere kleiner 5,0 Vol.-%, ist, wobei "Teilchen" in diesem Zusammenhang insbesondere Teilchen des Verbundpulvers im Sinne der Erfindung, kleine Teilchen im Sinne der Erfindung sowie Bruchstücke oder Fragmente der großen und/oder der kleinen Teilchen im Sinne der Erfindung umfassen, sofern sie die genannte Teilchengröße aufweisen.

Ferner ist die Dichte des Feinkornanteils vorzugsweise kleiner 2,6 g/cm³, bevorzugt kleiner 2,5 g/cm³, besonders bevorzugt kleiner 2,4 g/cm³, insbesondere im Bereich von größer 1,2 g/cm³ bis kleiner 2,4 g/cm³, wobei dieser Wert vorzugsweise durch Abtrennung des Feinkornanteils mittels Sieben und Dichtemessung an der abgetrennten Fraktion bestimmt wird.

Bevorzugt weisen die Teilchen des Verbundpulvers eine Teilchengröße d₉₀ von kleiner 350 µm, vorzugsweise kleiner 300 µm, bevorzugt kleiner 250 um, besonders bevorzugt kleiner 200 µm, insbesondere kleiner 150 µm auf. Weiterhin ist die Teilchengröße d₉₀ vorzugsweise größer 50 µm, bevorzugt größer 75 µm, insbesondere größer 100 µm.

Zweckmäßigerweise ist das Verhältnis d₂₀/d₅₀ kleiner 100%, vorzugsweise kleiner 75%, bevorzugt kleiner 65%, besonders bevorzugt kleiner 60%, insbesondere kleiner 55%. Weiterhin ist das Verhältnis d₂₀/d₅₀ zweckmäßigerweise größer 10%, vorzugsweise größer 20%, bevorzugt größer 30%, besonders bevorzugt größer 40%, insbesondere größer 50%.

Die vorstehend genannten Größen d₂₀, dso und d₉₀ sind im Rahmen der vorliegenden Erfindung wie folgt definiert:
d₂₀ bezeichnet die Teilchengröße der Teilchengrößenverteilung, bei welcher 20% der Teilchen eine Teilchengröße kleiner als der angegebene Wert und 80% der Teilchen eine Teilchengröße größer als oder gleich dem angegebenen Wert haben.
dso bezeichnet die mittlere Teilchengröße der Teilchengrößenverteilung. 50% der Teilchen haben eine Teilchengröße kleiner als der angegebenen Wert und 50% der Teilchen haben eine Teilchengröße größer als oder gleich dem angegebenen Wert.
d₉₀ bezeichnet die Teilchengröße der Teilchengrößenverteilung, bei welcher 90% der Teilchen eine Teilchengröße kleiner als der angegebene Wert und 10% der Teilchen eine Teilchengröße größer als oder gleich dem angegebenen Wert haben.

Die Teilchengrößenverteilung dieser Ausführungsform kann auf an sich bekannte Weise durch Klassieren des Verbundpulvers erreicht werden, d.h. durch Trennen eines dispersen Feststoffgemischs in Fraktionen. Vorzugsweise erfolgt eine Klassierung nach der Partikelgröße oder Partikeldichte. Besonders vorteilhaft sind die Trockensiebung, die Nasssiebung und die Luftstrahlsiebung, insbesondere die Luftstrahlsiebung, sowie das Stromklassieren, insbesondere mittels Windsichten.

Im Rahmen einer besonders bevorzugten Ausführungsform der vorliegenden Erfindung wird das Verbundpulver in einem ersten Schritt klassiert, um die Grobfraktion größer 800 µm, bevorzugt größer 500 µm, insbesondere größer 250 µm, möglichst zu entfernen. In diesem Zusammenhang hat sich ein Trockensieben über ein grobes Sieb besonders bewährt, welches vorzugsweise eine Größe, gemeint sind die Größe der Öffnungen, im Bereich von 250 µm bis 800 µm, bevorzugt im Bereich von 250 µm bis 500 µm, insbesondere von 250 µm, aufweist.

In einem weiteren Schritt wird das Verbundpulver vorzugsweise klassifiziert, um den Feinanteil <20 µm möglichst zu entfernen. In diesem Zusammenhang haben Luftstrahlsiebung und Windsichten als besonders günstig erweisen.

Die mittleren Durchmesser der Teilchen des Verbundpulvers, der großen Teilchen und der kleinen Teilchen, die Teilchengrößen d₂₀, d₅₀, d₉₀ sowie die vorstehend genannten Längengrößen werden erfindungsgemäß zweckmäßigerweise anhand mikroskopischer Aufnahmen, ggf. anhand elektronenmikroskopischer Aufnahmen, ermittelt. Für die Ermittlung der mittleren Durchmesser der großen Teilchen und der kleinen Teilchen sowie der Teilchen des Verbundpulvers und für die Teilchengrößen d₂₀, d₅₀, d₉₀ sind auch Sedimentationsanalysen besonders vorteilhaft, wobei hier die Verwendung eines Sedigraphs 5100 (Micromeritics GmbH) besonders günstig ist. Für die Teilchen des Verbundpulvers haben sich auch Partikelgrößenanalysen mit Laserbeugung besonders bewährt, wobei in diesem Zusammenhang die Verwendung eines Laserbeugungssensors HELOS/F der Firma Sympatec GmbH besonders vorteilhaft ist. Dieser umfasst vorzugsweise einen RODOS Trockendispergierer.

Im Übrigen beziehen sich diese Angaben sowie alle anderen Angaben in der vorliegenden Beschreibung, sofern nichts anderes angegeben wird, auf eine Temperatur von 23°C.

Das erfindungsgemäße Verbundpulver ist vergleichsweise kompakt. Vorzugsweise ist der Anteil von Teilbereichen im Inneren der Teilchen des Verbundpulvers, die eine Dichte kleiner 0,5 g/cm³, insbesondere kleiner 0,25 g/cm³, aufweisen, kleiner 10,0 %, bevorzugt kleiner 5,0 %, insbesondere kleiner 1,0 %, jeweils bezogen auf das Gesamtvolumen des Verbundpulvers.

Der Gewichtsanteil der kugelförmigen Calciumcarbonat-Teilchen, bezogen auf das Gesamtgewicht des Verbundpulvers, beträgt vorzugsweise mindestens 0,1 Gew.-%, bevorzugt mindestens 1,0 Gew.-%, besonders bevorzugt mindestens 5,0 Gew.-%, und liegt zweckmäßigerweise im Bereich von 5,0 Gew.-% bis 80,0 Gew.-%, besonders bevorzugt im Bereich von 10,0 Gew.-% bis 60,0 Gew.-%, günstigerweise im Bereich von 20,0 Gew.-% bis 50,0 Gew.-%. Für kugelförmige Calciumcarbonat-Teilchen, welche, bezogen auf die Gesamtmenge an kugelförmigen Calciumcarbonat-Teilchen, mehr als 15,0 Gew.-% Teilchen mit einer Größe kleiner 20 µm und/oder Teilchen mit einer Größe größer 250 µm enthalten, hat sich eine Gesamtmenge an kugelförmigen Calciumcarbonat-Teilchen im Bereich von 35,0 Gew.-% bis 45,0 Gew.-% ganz besonders bewährt. Für kugelförmige Calciumcarbonat-Teilchen, welche, bezogen auf die Gesamtmenge an kugelförmigen Calciumcarbonat-Teilchen, höchstens 15,0 Gew.-% Teilchen mit einer Größe kleiner 20 µm und/oder Teilchen mit einer Größe größer 250 µm enthalten, hat sich eine Gesamtmenge an kugelförmigen Calciumcarbonat-Teilchen im Bereich von 20,0 Gew.-% bis 30,0 Gew.-% ganz besonders bewährt.

Der Gewichtsanteil des Polymers, bevorzugt des thermoplastischen Polymers, bezogen auf das Gesamtgewicht des Verbundpulvers, beträgt vorzugsweise mindestens 0,1 Gew.-%, bevorzugt mindestens 1,0 Gew.-%, besonders bevorzugt mindestens 5,0 Gew.-%, und liegt zweckmäßigerweise im Bereich von 20,0 Gew.-% bis 95,0 Gew.-%, im Bereich von 40,0 Gew.-% bis 90,0 Gew.-%, günstigerweise im Bereich von 50,0 Gew.-% bis 80,0 Gew.-%.

Für ein Verbundpulver, das kugelförmige Calciumcarbonat-Teilchen enthält, welche, bezogen auf die Gesamtmenge an kugelförmigen Calciumcarbonat-Teilchen, mehr als 20,0 Gew.-% Teilchen mit einer Größe kleiner 20 µm und/oder Teilchen mit einer Größe größer 250 µm enthalten, hat sich eine Gesamtmenge an Polymer im Bereich von 55,0 Gew.-% bis 65,0 Gew.-% ganz besonders bewährt. Für ein Verbundpulver, das kugelförmige Calciumcarbonat-Teilchen enthält, welche, bezogen auf die Gesamtmenge an kugelförmigen Calciumcarbonat-Teilchen, höchstens 20,0 Gew.-% Teilchen mit einer Größe kleiner 20 µm und/oder Teilchen mit einer Größe größer 250 µm enthalten, hat sich eine Gesamtmenge an Polymer im Bereich von 70,0 Gew.-% bis 80,0 Gew.-% ganz besonders bewährt.

Das Verbundpulver zeichnet sich unter anderem durch eine sehr gute Verbindung des ersten Materials mit dem zweiten Material aus. Die feste Verbindung des ersten Materials mit dem zweiten Material lässt sich vorzugsweise durch mechanische Beanspruchung des Verbundpulvers, insbesondere durch Ausschütteln des Verbundpulvers mit Nichtlösungsmittel für das Polymer und kugelförmige Calciumcarbonat bei 25°C, vorzugsweise gemäß der in Organikum, 17. Auflage, VEB Deutscher Verlag der Wissenschaften, Berlin, 1988, Abschnitt 2.5.2.1 "Ausschütteln von Lösungen bzw. Suspensionen", Seite 56-57 beschriebenen Vorgehensweise, verifizieren. Die Schüttelzeit beträgt vorzugsweise mindestens eine Minute, bevorzugt mindestens 5 Minuten, insbesondere 10 Minuten, und führt vorzugsweise nicht zu einer wesentlichen Veränderung der Form, der Größe und/oder der Zusammensetzung der Teilchen des Verbundpulvers. Besonders bevorzugt sind nach dem Schütteltest mindestens 60 Gew.-%, vorzugsweise mindestens 70 Gew.-%, bevorzugt mindestens 80 Gew.-%, besonders bevorzugt mindestens 90 Gew.-%, günstigerweise mindestens 95 Gew.-%, insbesondere mindestens 99 Gew.-% der Teilchen des Verbundpulvers, hinsichtlich ihrer Zusammensetzung, ihrer Größe und vorzugsweise ihrer Form nicht verändert. Ein in diesem Zusammenhang besonders geeignete Nicht-Lösungsmittel ist Wasser, insbesondere für Polyamid enthaltendes Verbundpulver.

Weiterhin weisen die Teilchen des erfindungsgemäßen Verbundpulvers üblicherweise eine vergleichsweise isotrope Partikelform auf, die insbesondere für Anwendungen des Verbundpulvers in SLM-Verfahren vorteilhaft ist. Die gewöhnlich nahezu kugelförmige Teilchenform der Teilchen des Verbundpulvers führt in der Regel zu einer Vermeidung oder zumindest zu einer Reduzierung von negativen Einflüssen, wie Verzug oder Schwindung. Infolgedessen ist üblicherweise auch ein sehr vorteilhaftes Aufschmelz- und Erstarrungsverhalten des Verbundpulvers zu beobachten.

Im Gegensatz dazu haben herkömmliche Pulverpartikel, die beispielsweise durch kryogenes Mahlen erhalten werden, eine unregelmäßige (amorphe) Teilchenform mit scharfen Kanten und spitzen Ecken. Derartige Pulver sind jedoch wegen ihrer nachteiligen Teilchenform sowie zusätzlich wegen ihrer vergleichsweise breiten Teilchengrößenverteilung und wegen ihrem vergleichsweise hohen Feinanteil von Teilchen <20 µm für SLM-Verfahren nicht von Vorteil.

Durch die Calciumcarbonat-Teilchen, insbesondere durch die gefällten Calciumcarbonat-Teilchen, können die Eigenschaften des Polymers, insbesondere des thermoplastischen Polymers, gezielt beeinflusst und gesteuert werden. So ermöglichen die Calciumcarbonat-Teilchen, insbesondere die gefällten Calciumcarbonat-Teilchen, eine sehr gute Pufferung und pH-Stabilisierung des Polymers, insbesondere des thermoplastischen Polymers. Darüber hinaus wird die Biokompatibilität des Polymers, insbesondere des thermoplastischen Polymers, durch die Calciumcarbonat-Teilchen, insbesondere durch die gefällten Calciumcarbonat-Teilchen, deutlich verbessert. Darüber hinaus wird bei Verwendung der inhibierenden Calciumcarbonat-Teilchen eine deutliche Unterdrückung des thermischen Abbaus des Polymers, insbesondere des thermoplastischen Polymers, beobachtet.

Die Herstellung des erfindungsgemäßen Verbundpulvers kann auf an sich bekannte Weise, beispielsweise durch ein einstufiges Verfahren, insbesondere durch Auffällen oder Beschichten, vorzugsweise durch Beschichten mit Mahlgut, erfolgen. Weiterhin ist auch eine Vorgehensweise besonders geeignet, bei welcher man Polymerteilchen aus einer Polymerlösung ausfällt, die zusätzlich kleine Teilchen im Sinne der Erfindung, vorzugsweise in suspendierter Form enthält.

Besonders bewährt hat sich jedoch eine Vorgehensweise, bei welcher man Polymerpartikel und kugelförmige Calciumcarbonat-Teilchen miteinander in Kontakt bringt und durch Einwirkung mechanischer Kräfte miteinander verbindet. Zweckmäßigerweise erfolgt dies in einem geeigneten Mischer oder in einer Mühle, insbesondere in einer Prallmühle, Stiftmühle oder in einer Ultrarotormühle. Die Rotorgeschwindigkeit ist dabei vorzugsweise größer 1 m/s, bevorzugt, größer 10 m/s, besonders bevorzugt größer 25 m/s, insbesondere im Bereich von 50 m/s bis 100 m/s.

Die Temperatur, bei welcher die Herstellung des Verbundpulvers erfolgt, kann grundsätzlich frei gewählt werden. Besonders vorteilhaft sind jedoch Temperaturen größer -200°C, vorzugsweise größer -100°C, bevorzugt größer - 50°C, besonders bevorzugt größer -20°C, insbesondere größer 0°C. Andererseits ist die Temperatur vorteilhafterweise kleiner 120°C, vorzugsweise kleiner 100°C, bevorzugt kleiner 70°C besonders bevorzugt kleiner 50°C, insbesondere kleiner 40°C. Ganz besonders bewährt haben sich Temperaturen im Bereich von größer 0°C bis kleiner 50°C, insbesondere im Bereich von größer 5°C bis kleiner 40°C.

Im Rahmen einer besonders bevorzugten Ausführungsform der vorliegenden Erfindung wird der Mischer oder die Mühle, insbesondere die Prallmühle, die Stiftmühle oder die Ultrarotormühle, während der Herstellung des erfindungsgemäßen Verbundpulvers gekühlt, um die freiwerdende Energie abzuführen. Vorzugsweise erfolgt eine Kühlung mit einem Kühlmedium, das eine Temperatur kleiner 25°C, bevorzugt im Bereich von kleiner 25°C bis -60°C, besonders bevorzugt im Bereich von kleiner 20°C bis -40°C, zweckmäßigerweise im Bereich von kleiner 20°C bis -20°C, insbesondere im Bereich von kleiner 15°C bis 0°C, aufweist. Weiterhin ist die Kühlung vorzugsweise derart dimensioniert, dass am Ende des Misch- oder Mahlvorgangs, bevorzugt des Mahlvorgangs, die Temperatur im Misch- oder Mahlraum, bevorzugt im Mahlraum, kleiner 120°C, vorzugsweise kleiner 100°C, bevorzugt kleiner 70°C besonders bevorzugt kleiner 50°C, insbesondere kleiner 40°C, ist.

Gemäß einer besonders bevorzugten Ausführungsform der vorliegenden Erfindung führt diese Vorgehensweise insbesondere bei Polyamiden dazu, dass die kugelförmigen Calciumcarbonat-Teilchen in das Innere der Polymerpartikel eindringen und von dem Polymer möglichst vollständig bedeckt werden, so dass sie von außen nicht erkennbar sind. Derartige Teilchen können wie das Polymer ohne die kugelförmigen Calciumcarbonat-Teilchen verarbeitet und verwendet werden, weisen aber die verbesserten Eigenschaften des erfindungsgemäßen Verbundpulvers auf.

Im Rahmen einer ersten besonders bevorzugten Variante der vorliegenden Erfindung erfolgt die Herstellung des Verbundpulvers in Anlehnung an die in der Patentanmeldung JP62083029 A beschriebenen Vorgehensweise. Dabei wird ein erstes Material (sogenannte Mutterpartikel) mit einem zweiten Material, das aus kleineren Partikeln (sogenannte Babypartikel) besteht, auf der Oberfläche beschichtet. Zu diesem Zweck wird vorzugsweise eine Oberflächenmodifizierungsvorrichtung (*"Hybridizer"*) eingesetzt, die einen Hochgeschwindigkeitsrotor, einen Stator und ein kugelförmiges Gefäß, vorzugsweise umfassend innenliegende Messer, umfasst. Der Einsatz von NARA Hybridization Systemen, die vorzugsweise einen Rotor-Außendurchmesser von 118 mm aufweisen, insbesondere von einem Hybridization System mit der Bezeichnung NHS-0 oder NHS-1 der Firma NARA Machinery Co., Ltd., hat sich in diesem Zusammenhang besonders bewährt.

Die Mutterpartikel und die Babypartikel werden gemischt, vorzugsweise feinstverteilt und in den Hybridizer eingebracht. Dort wird die Mischung vorzugsweise weiter feinstverteilt und vorzugsweise wiederholt mechanischen Kräften ausgesetzt, insbesondere Stoßkräften, Kompressionskräften, Reibungskräften und Scherkräften sowie den gegenseitigen Wechselwirkungen der Teilchen, um die Babyteilchen in den Mutterteilchen einheitlich einzubetten. Bevorzugte Rotorgeschwindigkeiten liegen im Bereich von 50 m/s bis 100 m/s, bezogen auf die Umfangsgeschwindigkeit.

Für weitere Details zu diesem Verfahren, insbesondere bezüglich der besonders zweckmäßigen Ausführungsformen, wird auf die JP62083029 A verwiesen. Im Rahmen einer weiteren besonders bevorzugten Variante der vorliegenden Erfindung erfolgt die Herstellung des Verbundpulvers in Anlehnung an die in der Patentanmeldung DE 42 44 254 A1 beschriebenen Vorgehensweise. Dementsprechend ist ein Verfahren zur Herstellung eines Verbundpulvers durch Befestigen einer Substanz auf der Oberfläche eines thermoplastischen Materials besonders günstig, wenn das thermoplastische Material einen durchschnittlichen Teilchendurchmesser von 100 µm bis 10 mm hat und die Substanz einen geringeren Teilchendurchmesser und eine bessere Hitzebeständigkeit hat als das thermoplastische Material, insbesondere wenn das Verfahren die Schritte umfasst:
∘ zuerst Erhitzen der Substanz, die den geringeren Teilchendurchmesser und die bessere Hitzebeständigkeit hat als das thermoplastische Material, auf eine Temperatur, die vorzugsweise nicht kleiner ist als der Erweichungspunkt des thermoplastischen Materials, unter Rühren in einer Vorrichtung, die vorzugsweise ein Rührwerk und eine Heizeinrichtung hat;
∘ Zugeben des thermoplastischen Materials in die Vorrichtung; und
∘ Befestigen der Substanz mit der besseren Hitzebeständigkeit auf der Oberfläche des thermoplastischen Materials.

Für weitere Details zu diesem Verfahren, insbesondere bezüglich der besonders zweckmäßigen Ausführungsformen, wird auf die DE 42 44 254 A1 verwiesen.

Im Rahmen noch einer weiteren besonders bevorzugten Variante der vorliegenden Erfindung erfolgt die Herstellung des Verbundpulvers in Anlehnung an die in der Patentanmeldung EP 0 922 488 A1 und/oder in dem Patent US 6,403,219 B1 beschriebenen Vorgehensweise. Dementsprechend ist ein Verfahren zur Herstellung eines Verbundpulvers durch Befestigung oder Ankleben von feinen Partikeln auf der Oberfläche eines Feststoffpartikels, das als ein Kern fungiert, durch Anwendung eines Aufpralls und anschließendes Wachstum von einem oder mehreren Kristallen auf der Kernoberfläche besonders vorteilhaft.

Für weitere Details zu diesem Verfahren, insbesondere bezüglich der besonders zweckmäßigen Ausführungsformen, wird auf die Patentanmeldung EP 0 922 488 A1 und/oder das Patent US 6,403,219 B1 verwiesen.

Im Rahmen einer weiteren besonders bevorzugten Ausführungsform der vorliegenden Erfindung wird das Verbundpulver einer Fixierung in Anlehnung an die in der Patentanmeldung EP 0 523 372 A1 beschriebenen Vorgehensweise unterzogen. Diese Vorgehensweise ist insbesondere für ein Verbundpulver zweckmäßig, das in Anlehnung an das in der Patentanmeldung JP62083029 A beschriebene Verfahren erhalten wurden. Die Fixierung der Teilchen des Verbundpulvers erfolgt dabei vorzugsweise durch thermisches Plasmaspritzen, wobei vorzugsweise eine Unterdruckplasmasprühvorrichtung ("reduced pressure plasma spraying device") eingesetzt wird, die vorzugsweise eine Leistung von mindestens 30 kW aufweist, insbesondere das in EP 0 523 372 A1 beschriebene Gerät.

Für weitere Details zu diesem Verfahren, insbesondere bezüglich der besonders zweckmäßigen Ausführungsformen, wird auf die Patentanmeldung EP 0 523 372 A1 verwiesen.

Das erfindungsgemäße Verbundpulver zeichnet sich durch ein hervorragendes Eigenschaftsprofil aus, welche ihre Verwendung insbesondere in Lasersinterverfahren nahelegt. Ihre hervorragende Rieselfähigkeit und ihre exzellente Fließfähigkeit ermöglichen beim Lasersintern die Herstellung von Bauteilen mit hervorragender Oberflächengüte und Oberflächenbeschaffenheit sowie verbesserter Bauteildichte. Gleichzeitig zeigt das erfindungsgemäße Verbundpulver ein sehr gutes Schrumpfverhalten sowie eine hervorragende Dimensionsstabilität. Weiterhin ist ein besseres Wärmeleitverhalten außerhalb des laserbehandelten Bereichs festzustellen.

Weiterhin weist das erfindungsgemäße Verbundpulver eine vergleichsweise hohe Isotropie auf, die ein überaus gleichmäßiges Aufschmelzen des Verbundpulvers ermöglicht. Dieses Verhalten kann in SLM-Verfahren zur Herstellung von Bauteilen mit hoher Qualität, hoher Bauteildichte, geringer Porosität und geringer Anzahl von Fehlstellen genutzt werden.

Weiterhin ermöglicht die Anwesenheit der kugelförmigen Calciumcarbonat-Teilchen im Verbundpulver eine hervorragende pH-Wert-Stabilisierung (Pufferung) in späteren Anwendungen, insbesondere in solchen Polymeren, die Säuregruppen enthalten oder unter bestimmten Bedingungen Säuren freisetzen können. Hierzu gehören beispielsweise Polyvinylchlorid und Polymilchsäure.

Ferner können durch das erfindungsgemäße Verbundpulver ggf. andere, teurere Werkstoffe ersetzt werden, um so eine Verbilligung des Endprodukts zu erreichen.

Erfindungsgemäß können die Eigenschaften des Verbundpulvers, insbesondere seine Fließfähigkeit, auch über die Feuchtigkeit des Verbundpulvers gesteuert und bei Bedarf gezielt eingestellt werden. Einerseits nimmt die Fließfähigkeit des Verbundpulvers mit zunehmender Feuchtigkeit grundsätzlich zu, welches die Verarbeitbarkeit des Verbundpulvers erleichtert. Andererseits kann eine höhere Feuchtigkeit des Verbundpulvers insbesondere bei thermischer Verarbeitung des Verbundpulvers vor allem in Gegenwart von Verunreinigungen und/oder dem Vorliegen sehr feiner Teilchen zu thermischen Abbau oder Hydrolyse des Polymers sowie zu Prozessstörungen führen.

Vor diesem Hintergrund ist die Feuchtigkeit des erfindungsgemäßen Verbundpulvers vorzugsweise kleiner 2,5 Gew.-%, bevorzugt kleiner 1,5 Gew.-%, besonders bevorzugt kleiner 1,0 Gew.-%, noch mehr bevorzugt kleiner 0,9 Gew.-%, günstigerweise kleiner 0,8 Gew.-%, zweckmäßigerweise kleiner 0,6 Gew.-%, ganz besonders bevorzugt kleiner 0,5 Gew.-%, insbesondere kleiner 0,25 Gew.-%. Andererseits ist die Feuchtigkeit des erfindungsgemäßen Verbundpulvers vorzugsweise größer 0,000 Gew.-%, bevorzugt größer 0,010 Gew.-%, insbesondere größer 0,025 Gew.-%.

Die Verwendung des inhibierenden Calciumcarbonats ermöglicht in diesem Zusammenhang eine nochmals verbesserte thermische Verarbeitbarkeit des Verbundpulvers. Das Verarbeitungsfenster (Temperaturfenster) wird nochmals deutlich größer als mit herkömmlichen Calciumcarbonat und ein thermischer Abbau oder eine Hydrolyse eines Polymers wird nochmals signifikant unterdrückt.

Die gewünschte Feuchtigkeit des Verbundpulvers kann durch an sich bekannte Vortrocknung des Verbundpulvers vor der Verarbeitung erreicht werden, wobei eine Trocknung im Produktionsprozess grundsätzlich zu empfehlen ist. Für eine stabile Prozessführung hat sich in diesem Zusammenhang eine Trocknung bis zu einem Feuchtegehalt im Bereich von 0,01 Gew.-% bis 0,1 Gew.-% als ganz besonders günstig erwiesen. Weiterhin hat sich die Verwendung eines Mikrowellen-Vakuumtrockners ganz besonders bewährt.

Die Weiterverarbeitung des Verbundpulvers kann auf vergleichbar einfache Art und Weise erfolgen, da nach der erfindungsgemäßen Lösung nur eine Komponente (das Verbundpulver) und nicht mehr zwei Komponenten (kugelförmige Calciumcarbonat-Teilchen und Polymer) zu verarbeiten sind. Dispergierprobleme sind aufgrund der festen Verbindung zwischen dem Polymer und den kugelförmigen Calciumcarbonat-Teilchen nicht zu beobachten.

Weiterhin kann über die Wahl der Anteile und der Größe der jeweiligen Einzelkomponenten die Mikrostruktur, das Schmelzverhalten und das Fließverhalten des Verbundpulvers gezielt gesteuert werden. Diese Eigenschaften des Verbundpulver können wiederum genutzt werden, um die Endstruktur der resultierenden Bauteile, insbesondere ihre Bioverträglichkeit, ihre Bioabbaubarkeit und ihre mechanischen Eigenschaften, gezielt zu steuern.

Ein Zusatz von weiteren Verarbeitungshilfsmitteln, insbesondere von speziellen Lösungsmitteln, ist bei der Verarbeitung des Verbundpulvers in der Regel nicht erforderlich. Dies erweitert die möglichen Anwendungsgebiete des Verbundpulvers insbesondere im Pharma- und im Nahrungsmittelsektor.

Das Verbundpulver kann als solches direkt eingesetzt werden. Aufgrund seines hervorragenden Eigenschaftsprofils eignet sich das Verbundpulver jedoch insbesondere als Additiv, besonders bevorzugt als Polymeradditiv, als Zusatzstoff oder Ausgangsmaterial für Compoundierung, für die Herstellung von Bauteilen, für Anwendungen in der Medizintechnik und/oder in der Mikrotechnik und/oder für die Herstellung von geschäumten Gegenständen. Besonders bevorzugte medizintechnische Anwendungen schließen vorzugsweise resorbierbare Implantate ein. Besonders zweckmäßige Anwendungsgebiete umfassen spritzgegossene Schrauben, gepresste Platten, insbesondere schmelzgepresste Platten, geschäumte Implantate sowie rieselfähige Pulver für selektive Fertigungsverfahren, wobei im letzteren Fall die Gesamtteilchengröße der Teilchen des Verbundpulvers vorzugsweise kleiner 3 mm und vorzugsweise größer 5,0 µm ist.

Als Polymeradditiv wird das Verbundpulver vorzugsweise mindestens einem Polymer, insbesondere einem thermoplastischen Polymer, als Matrixpolymer zugesetzt. Besonders bevorzugt werden hier die Polymere, die auch als Komponente des Verbundpulvers verwendet werden können. Um Wiederholungen zu vermeiden, wird daher auf die obigen Ausführungen verwiesen, insbesondere hinsichtlich der bevorzugten Formen des Polymers. Ganz besonders bevorzugte Matrixpolymere schließen Polyvinylchlorid (PVC), Polyurethan (PU), Silikon, Polypropylen (PP), Polyethylen (PE), insbesondere UHMWPE, und Polymilchsäure (PLA) ein.

Im Rahmen der vorliegenden Erfindung sind das Matrixpolymer und das Polymer des Verbundpulvers vorzugsweise bei der Anwendungstemperatur miteinander mischbar, besonders bevorzugt chemisch identisch.

Besonders bevorzugte Zusammensetzungen enthalten 40,0 Gew.-% bis 99,9 Gew.-% mindestens eines Matrixpolymers und 0,1 Gew.-% bis 50,0 Gew.-% mindestens eines erfindungsgemäßen Verbundpulvers.

Die Herstellung der Zusammensetzung kann auf an sich bekannte Weise durch Mischen der Komponenten erfolgen.

Die Zusammensetzung kann dann in üblicherweise weiterverarbeitet, insbesondere granuliert, gemahlen, extrudiert, spritzgegossen, geschäumt oder auch in 3D-Druckverfahren verwendet werden.

Weiterhin kann das Verbundpulver direkt, d. h. ohne Zusatz von zusätzlichen Polymeren, weiterverarbeitet und/oder verwendet werden.

Die Vorteile des Verbundpulvers sind dabei insbesondere beim Granulieren, Mahlen, Extrudieren, Spritzgießen, Schmelzpressen, Schäumen und/oder 3D-Drucken des Verbundpulvers zu beobachten.

Die Herstellung von Polymerschäumen erfolgt vorzugsweise durch die Erzeugung oder Einbringung einer gasförmigen Phase in eine Zusammensetzung, umfassend das Verbundpulver und ggf. mindestens ein Matrixpolymer. Ziel ist es dabei, das Gas möglichst gleichmäßig in der Zusammensetzung zu verteilen, um eine gleichmäßige und homogene Schaumstruktur zu erreichen. Die Einbringung des Gases kann auf verschiedene Weise erfolgen.

Bevorzugt erfolgt die Erzeugung der Gasphase durch Zugabe eines Treibmittels. Als Treibmittel werden Substanzen bezeichnet, die durch chemische Reaktionen (chemische Treibmittel) oder durch Phasenübergang (physikalische Treibmittel) Gase freisetzen. Bei der Schaumextrusion oder beim Schaumspritzguss wird das chemische Treibmittel in Form eines Masterbatches der Zusammensetzung beigemengt oder physikalisches Treibmittel direkt in die Schmelze der Zusammensetzung unter Druck injiziert. Die Injektion wird als Direktbegasung bezeichnet und findet insbesondere bei der Verarbeitung von thermoplastischen Polymeren Einsatz.

Darüber hinaus eignet sich das erfindungsgemäße Verbundpulver insbesondere zur Herstellung von Implantaten, die herkömmliche Implantate aus Metall bei Knochenbrüchen ersetzen können. Die Implantate dienen der Fixierung der Knochen bis zur Heilung des Bruchs. Während Implantate aus Metall normalerweise im Körper verbleiben oder in einer weiteren Operation entfernt werden müssen, fungieren die aus dem erfindungsgemäßen Verbundpulver erhältlichen Implantate als Helfer auf Zeit. Sie umfassen zweckmäßigerweise Polymere, die der Körper selbst abbauen kann, und Substanzen, die Calcium und vorzugsweise wertvolle Phosphorsubstanzen für den Knochenaufbau liefern. Die sich ergebenden Vorteile für den Patienten sind klar: keine weitere Operation zur Entfernung des Implantats und eine beschleunigte Regeneration der Knochen.

Gemäß einer besonders bevorzugten Variante der vorliegenden Erfindung wird das erfindungsgemäße Verbundpulver zur Herstellung von Bauteilen, insbesondere Implantaten durch selektives Lasersintern eingesetzt. Zweckmäßigerweise werden zu einem Pulverbett dicht nebeneinander gepackte Teilchen des erfindungsgemäßen Verbundpulvers mithilfe einer Laser-Scanner-Einheit, eines direkt abgelenkten Elektronenstrahls oder einer Infrarot-Heizung mit einer die Geometrie abbildenden Maske örtlich leicht an- oder aufgeschmolzen (nur das Polymer). Sie erstarren durch Abkühlung infolge von Wärmeleitung und verbinden sich so zu einer festen Schicht. Die nicht angeschmolzenen Pulverkörnchen verbleiben als Stützmaterial im Bauteil und werden nach Beendigung des Bauprozesses vorzugsweise entfernt. Durch erneutes Beschichten mit Pulver können in Analogie zur ersten Schicht weitere Schichten verfestigt und mit der ersten Schicht verbunden werden.

Für Lasersinterverfahren besonders geeignete Lasertypen sind alle, die das Polymer des erfindungsgemäßen Verbundpulvers zum Versintern, Verschmelzen oder Vernetzen bringen, insbesondere CO₂ Laser (10 µm) ND-YAG Laser (1.060 nm), He-Ne-Laser (633 nm) oder Farbstofflaser (350-1.000 nm). Bevorzugt wird ein CO₂ Laser verwendet.

Die Energiedichte in der Schüttung beträgt bei der Bestrahlung vorzugsweise von 0,1 J/mm³ bis 10 J/mm³.

Der Wirkdurchmesser des Laserstrahles beträgt vorzugweise je nach Anwendung von 0,01 nm bis 0,5 nm, bevorzugt 0,1 nm bis 0,5 nm.

Bevorzugt werden gepulste Laser verwendet, wobei sich eine hohe Pulsfrequenz, insbesondere von 1 kHz bis 100 kHz, als besonderes geeignet erwiesen hat.

Die bevorzugte Verfahrensführung lässt sich wie folgt beschreiben:
Der Laserstrahl trifft auf die oberste Schicht der Schüttung aus dem erfindungsgemäß einzusetzenden Material und versintert dabei das Material in einer bestimmten Schichtdicke. Diese Schichtdicke kann von 0,01 mm bis 1 mm, vorzugsweise von 0,05 mm bis 0,5 mm betragen. Auf diese Weise wird die erste Schicht des gewünschten Bauteiles erzeugt. Anschließend wird der Arbeitsraum um einen Betrag abgesenkt, der niedriger ist als die Dicke der zusammengesinterten Schicht. Der Arbeitsraum wird bis zur ursprünglichen Höhe mit zusätzlichem Polymer-Material aufgefüllt. Durch erneute Bestrahlung mit dem Laser wird die zweite Schicht des Bauteils gesintert und mit der vorhergegangenen Schicht verbunden. Durch Wiederholung des Vorgangs werden die weiteren Schichten bis zur Fertigstellung des Bauteils erzeugt.

Die Belichtungsgeschwindigkeit bei der Abtastung des Lasers beträgt vorzugsweise 1 mm/s bis 1.000 mm/s. Typischerweise wird eine Geschwindigkeit von etwa 100 mm/s angewendet.

Im vorliegenden Fall hat sich zum An- oder Aufschmelzen des Polymers eine Erwärmung auf eine Temperatur im Bereich von 60°C bis 250°C, bevorzugt im Bereich von 100°C bis 230°C, insbesondere im Bereich von 150°C bis 200°C besonders bewährt.

Gegenstand der vorliegenden Erfindung sind auch Bauteile, die durch selektives Lasersintern einer Zusammensetzung erhältlich sind, die ein erfindungsgemäßes Verbundpulver umfasst, wobei Implantate für Anwendungen im Bereich der Neuro-, Mund-, Kiefer-, Gesichts-, Hals-, Nasen- und Ohrenchirurgie sowie der Hand-, Fuß-, Thorax-, Rippen- und Schulterchirurgie als Bauteile ausgenommen werden.

Der Gewichtsanteil des erfindungsgemäßen Verbundpulvers in der Zusammensetzung beträgt vorzugsweise mindestens 50,0 Gew.-%, bevorzugt mindestens 75,0 Gew.-%, besonders bevorzugt mindestens 90,0 Gew.-%, insbesondere mindestens 99,0 Gew.-%. Im Rahmen einer ganz besonders Ausführungsform der vorliegenden Erfindung enthält die Zusammensetzung ausschließlich das erfindungsgemäße Verbundpulver.

Die erfindungsgemäßen Bauteile zeichnen sich günstigerweise durch die folgenden Eigenschaften aus:
- hervorragende Oberflächengüte,
- hervorragende Oberflächenbeschaffenheit,
- hervorragende Bauteildichte, vorzugsweise größer 95%, insbesondere größer 97%,
- hervorragendes Schrumpfverhalten,
- hervorragende Dimensionsstabilität,
- sehr wenige Fehlstellen,
- sehr geringere Porosität,
- sehr geringer Gehalt an Abbauprodukten,
- hervorragende Drei-Punkt-Biegefestigkeit, bevorzugt größer 60 MPa, besonders bevorzugt größer 65 MPa, insbesondere größer 70 MPa,
- hervorragendes E-Modul, bevorzugt 3420 N/mm², besonders bevorzugt größer 3750 N/mm², günstigerweise größer 4000 N/mm², insbesondere größer 4500 N/mm²,
- hervorragende pH-Stabilität,
- hervorragende Bioverträglichkeit,
- hervorragende Biokompatibilität,
- hervorragende Osteokonduktion,
- hervorragende Resorbierbarkeit,
- hervorragende Bioabbaubarkeit.

Eine thermoplastische Weiterverarbeitung der erfindungsgemäßen Verbundteilchen bewirkt üblicherweise ein mindestens teilweise Verschmelzen der Verbundteilchen infolge des An- oder Aufschmelzens des in ihnen enthaltenen Polymers. Bevorzugt führt diese thermoplastische Weiterverarbeitung jedoch nicht zu einer homogenen Verteilung der kleinen Teilchen oder Fragmente davon auf der Oberfläche oder im Inneren der großen, nun verschmolzenen Teilchen, insbesondere da die kleinen Teilchen oder Fragmente davon unter den Weiterverarbeitungsbedingungen vorzugsweise weder an- noch aufschmelzen. Daher weisen die resultierenden Bauteile vorzugswiese eine vergleichbare Inhomogenität bezüglich der Verteilung der kleinen Teilchen oder Fragmente davon auf der Oberfläche oder im Inneren der nun verschmolzenen großen Teilchen auf, wenn man die Größe der weiterverarbeiteten Verbundteilchen als Größenmaßstab für die Bewertung zugrunde legt.

Gegenstand der Erfindung sind auch die kugelförmigen Calciumcarbonat-Teilchen, die zur Herstellung der erfindungsgemäßen Verbundteilchen vorteilhaft eingesetzt werden können sowie deren Verwendung.

Somit betriftt die vorliegende Erfindung auch kugelförmige Calciumcarbonat-Teilchen, die nach einem Verfahren erhältlich sind, bei welchem man
a. eine Calciumhydroxid-Suspension vorlegt,
b. in die Suspension aus Schritt a. Kohlendioxid oder eine Kohlendioxid-haltige Gasmischung einleitet und
c. entstehende Calciumcarbonat-Teilchen abtrennt,
wobei man weiterhin 0,3 Gew.-% bis 0,7 Gew.-% mindestens einer Aminotrialkylenphosphonsäure zugibt.

Bezüglich der bevorzugten Ausgestaltung dieser kugelförmigen Calciumcarbonat-Teilchen und bevorzugter Verfahren zu ihrer Herstellung gelten die obigen Ausführungen analog.

Bevorzugte Anwendungsgebiete der kugelförmigen Calciumcarbonat-Teilchen umfassen ihren Einsatz als Additiv für Papier, Kunststoffe, Farben und/oder Lacke, Elastomere sowie Kleb- und Dichtstoffe, in der Bauchemie, in Trockenmörtel und in der Medizintechnik, insbesondere als Additiv in resorbierbaren Polymeren.

Dabei sind insbesondere für Zusammensetzungen, umfassend, jeweils bezogen auf das Gesamtgewicht der Zusammensetzung,
a) mindestens 0,1 Gew.-%, bevorzugt mindestens 0,2 Gew.-%, insbesondere mindestens 0,5 Gew.-% bis 50,0 Gew.-%, mindestens eines kugelförmigen Calciumcarbonats und
b) mindestens 0,1 Gew.-%, bevorzugt mindestens 0,2 Gew.-%, insbesondere mindestens 0,5 Gew.-% bis 50,0 Gew.-%, mindestens eines Polymers, bevorzugt mindestens eines thermoplastischen Polymers, besonders bevorzugt mindestens eines resorbierbaren Polymers, insbesondere mindestens einer Poly-D-, Poly-L- und/oder Poly-D-L-milchsäure,
die in dieser Anmeldung genannten Vorteile und Effekte, insbesondere bzgl. der Verbesserung der mechanischen Eigenschaften und der Säurestabilität der Zusammensetzung, analog zu beobachten. Bezüglich der bevorzugten Wahl des Polymers gelten die obigen Ausführungen analog.

Im Folgenden wird die vorliegende Erfindung durch mehrere Beispiele und Vergleichsbeispiele weiter veranschaulicht, ohne dass hierdurch eine Beschränkung des Erfindungsgedankens erfolgen soll.
- Eingesetzte Materialien:
   - Granulat 1 (Poly(L-lactid); Inhärente Viskosität: 0,8-1,2 dl/g (0,1 % in Chloroform, 25°C); Tg: 60-65°C; Tm: 180-185°C)
   - Granulat 2 (Poly(L-lactid); Inhärente Viskosität: 1,5-2,0 dl/g (0,1 % in Chloroform, 25°C)); Tg: 60-65°C;
   - Granulat 3 (Poly(D,L-lactid); Inhärente Viskosität: 1,8-2,2 dl/g (0,1 % in Chloroform, 25°C)); Tg: 55-60°C; amorphes Polymer ohne Schmelzpunkt

Der mittlere Teilchendurchmesser der Polylactid-Granulate 1 bis 3 lag jeweils im Bereich von 1 bis 6 mm.

Im Rahmen der vorliegenden Beispiele wurden die folgenden Größen wie folgt ermittelt:
- CaCOs-Gehalt: Der CaC0₃-Gehalt wurde mittels Thermogravimetrie mit einem STA 6000 der Firma Perkin Elmer unter Stickstoff im Bereich von 40°C bis 1000°C bei einer Heizrate von 20°C/min ermittelt. Dabei wurde der Gewichtsverlust zwischen etwa 550°C und 1000°C bestimmt und daraus über den Faktor 2,274 (Molmassenverhältnis CaCOs : CO₂) der CaCOs-Gehalt in Prozent berechnet.
- β-Tricalciumphosphat-Gehalt (ß-TCP-Gehalt): Der β-TCP-Gehalt wurde mittels Thermogravimetrie mit einem STA 6000 der Firma Perkin Elmer unter Stickstoff im Bereich von 40°C bis 1000°C bei einer Heizrate von 20°C/min ermittelt. Der bei 1000°C verbliebene Gewichtsanteil entspricht dem β-TCP-Gehalt in Prozent.
- T_{P}: Die Peaktemperatur Tp wurde mittels Thermogravimetrie mit einem STA 6000 der Firma Perkin Elmer unter Stickstoff im Bereich von 40°C bis 1000°C bei einer Heizrate von 20°C/min ermittelt. Die Peaktemperatur der ersten Ableitung der Massenverlustkurve entspricht der Temperatur mit dem größten Massenverlust beim Polymerabbau.
- d₂₀, d₅₀, d₉₀: Die Bestimmung der Korngrößenverteilung des Calciumcarbonat-enthaltenden Verbundpulvers wurde mit Laserbeugung (HELOS Messbereich R5 mit RODOS-Dispergiersystem der Firma Sympatec) durchgeführt. Die Bestimmung der Korngrößenverteilung erfolgte für das Calciumcarbonat - Pulver mit dem Sedigraphen 5100 mit MasterTech 51 der Firma Micromeretics. Als Dispergierlösung wurde 0,1%ige Natriumpolyphosphatlösung (NPP) verwendet.
- Anteil <20 µm: Bestimmung analog zum dso. Auswertung des Anteils < 20 µm.
- Feuchte: Der Wassergehalt des Calciumcarbonat-enthaltenden Verbundpulvers wurde mittels Karl Fischer Coulometer C30 der Firma Mettler Toledo bei 150°C bestimmt. Der Wassergehalt der Calciumcarbonat-Pulver wurde mit dem Halogen-Feuchtigkeitsmessgerät HB43 von Mettler bei 130°C bestimmt (Einwaage: 6,4-8,6 g Pulver; Messdauer: 8 Minuten).
- Inhärente Viskosität: Die Inhärente Viskosität (dl/g) wurde mit einem Ubbelohde-Viskosimeter Kapillare 0c in Chloroform bei 25°C und 0,1 % Polymerkonzentration bestimmt.
- Fließfähigkeit: Die Beurteilung der Fließfähigkeit der Proben wurde mit einem elektromotorischen Filmziehgerät der Firma Erichsen durchgeführt. Hierzu wurde eine 200 µm bzw. 500 µm Rakel verwendet. Die Auftragsgeschwindigkeit auf den Folien-Typ 255 (Leneta) betrug 12,5 mm/s. Bewertung wie folgt: 1=sehr gut; 2=gut; 3=befriedigend; 4=ausreichend; 5=mangelhaft

Bestimmung der mechanischen Eigenschaften an spritzgegossenen Probekörpern:
Drei-Punkt-Biegefestigkeit und E-Modul wurden mittels Texture Analyser TA.XTplus (Stable Micro Systems, Godalming (UK)) bestimmt. Die Kapazität der verwendeten Kraftmesszelle betrug 50 kg. Es wurde Exponent 6.1.9.0 Software verwendet. Die Einzelheiten zur Messung sind in der folgenden Tabelle 1 dargestellt:

**Tabelle 1**

| | |
|---|---|
| Belastungseinrichtung: | Drei-Punkt-Belastung nach DIN EN 843-1 |
| | Durchmesser Auflager-/Belastungsrollen: 5,0 mm |
| Messung: | in Anlehnung an DIN EN ISO 178 |
| | Auflagerabstand: 45,0 mm |
| | Testgeschwindigkeit: 0,02 mm/s |
| | Vorgeschwindigkeit: 0,03 mm/s |
| | Aufzeichnung von Kraft und Weg |
| Probekörper: | Abmessungen ca. 3 mm x 10 mm x 50 mm nach der Herstellung (Spritzguss) Aufbewahrung bis zur Messung im Exsikkator bei Raumtemperatur n ≥ 5 |

Probekörper wurden mit dem Extruder HAAKE MiniLab II, bzw. Spritzguss mit dem HAAKE MiniJet II hergestellt. Die Prozessbedingungen zu der Probekörperherstellung sind in der folgenden Tabelle 2 zusammengefasst:

**Tabelle 2**

| Komposit | Temperatur Extruder [°C] | Temperatur Spritzguss [°C] | Temperatur Spritzguss-Form [°C] | Druck Spritzguss [bar] | Zeit Spritzguss [s] |
|---|---|---|---|---|---|
| Beispiel 3 | 180 | 180 | 80 | 700 | 10 |
| Beispiel 4 | 180 | 180 | 70 | 700 | 10 |
| Beispiel 5 | 185 | 185 | 80 | 700 | 10 |
| Beispiel 6 | 195 | 195 | 80 | 700 | 10 |
| Beispiel 7 | 175 | 175 | 72 | 700 | 10 |
| Vergleich 1 | 175 | 175 | 70 | 700 | 10 |

### Zytotoxizitätstest

Der Zytotoxizitätstest (FDA/GelRed) wurde wie folgt durchgeführt:
Als Referenz bzw. Negativkontrolle wurde Tissue Culture Polystyrene (TCPS) verwendet. Es wurden jeweils 4 Replikate pro Probe und vier TCPS (4x) als Kontrolle verwendet.

### Versuchsdurchführung:

1. Die unsterilen Proben wurden in einer 24 well - Mikrotiterplatte zur Verfügung gestellt. In dieser wurden die Proben sowie die TCPS-Plättchen 30 min mit 70%igem Ethanol (unvergällt) sterilisiert, anschließend 2 x 30 min mit 1 x PBS (Phosphatgepufferte Salzlösung) gespült und darauffolgend mit sterilem α-Medium equilibriert. Danach wurden die Proben mit MC3T3-E1-Zellen einer Animpfdichte von 25.000 Zellen/cm² (50.000 Zellen/ml) beimpft.
   Ein partieller Mediumsaustausch (1 : 2) erfolgte an Tag 2.
2. Nach 1 und 4 Tagen in Zellkultur wurde die Zytotoxizität bestimmt.
3. Die Vitalfärbung erfolgte am Tag 1 und 4 nach Standardprotokoll mittels einer Kombinationsfärbung aus FDA und GelRed.
4. Die mikroskopischen Aufnahmen wurden am Observer Z1 m/LSM 700 erzeugt. Objektiv: EC Plan-Neofluar 10x;
   Mit Kamera AxioCam HRc fotografierte Bilder:
      Anregung der Grünfluoreszenz: LED Colibri 470; Filtersatz FS10 (AF488)
      Anregung der Rotfluoreszenz: LED Colibri 530; Filtersatz FS14 (AF546)
   Im Laserscan-Modus erfasste Bilder:
      Track 1: Laser: 488 nm, DBS 560 nm, PMT1: 488 - 560 nm,
      Track 2: Laser: 555 nm, DBS 565 nm, PMT2: 565 - 800 nm
5. Die Bewertung erfolgte nach folgender Zytotoxizitätsskala: Akzeptanz: das Material ist nicht zytotoxisch (max. 5% tote Zellen) Leichte Hemmung: das Material ist schwach toxisch (5%-20% tote Zellen) Deutliche Hemmung: das Material ist mäßig toxisch (20%-50% tote Zellen) Toxizität: das Material ist stark zytotoxisch (>50%-100% tote Zellen)
6. Die Zellzahlen beziehen sich auf den fotografierten bzw. gescannten Bildausschnitt.

Die Ergebnisse sind in Tabelle 3 zusammengefasst.

### Elektronenmikroskop (REM)

Die rasterelektronischen Aufnahmen wurden mit einem Hochspannungselektronenmikroskop (Zeiss, DSM 962) bei 15 kV durchgeführt. Die Proben wurden mit einer Gold-Palladiumschicht besprüht.

### Beispiel 1 (Edukt für Verbundpulver gemäß der beanspruchten Erfindung)

In 4 l Calciumhydroxid-Suspension mit einer Konzentration von 75 g/l CaO wurde bei einer Ausgangstemperatur von 10°C ein CO₂-Gasgemisch, enthaltend 20% CO₂ und 80% N₂, eingeleitet. Der Gasdurchfluss betrug 300 l/h. Das Reaktionsgemisch wurde mit 350 U/min gerührt und die Reaktionswärme wurde während der Reaktion abgeführt. Beim abrupten Absinken des Leitwerts (Absinken von mehr als 0,5 mS/cm/min und Abnahme des Leitwerts um mehr als 0,25 mS/cm innerhalb von 30 Sekunden), wird der Suspension 0,7 % Aminotri(methylenphosphonsäure), bezogen auf CaO (als theoretische Bezugsgröße), zugegeben. Die Reaktion zu den kugelförmigen Calciumcarbonat-Teilchen war abgeschlossen, als das Reaktionsgemisch quantitativ zu kugelförmigen Calciumcarbonat-Teilchen carbonatisiert war, wobei die Reaktionsmischung dann einen pH-Wert zwischen 7 und 9 aufwies. Im vorliegenden Fall war die Reaktion nach ungefähr 2 h beendet und das Reaktionsgemisch wies zum Reaktionsende einen pH-Wert von 7 auf.

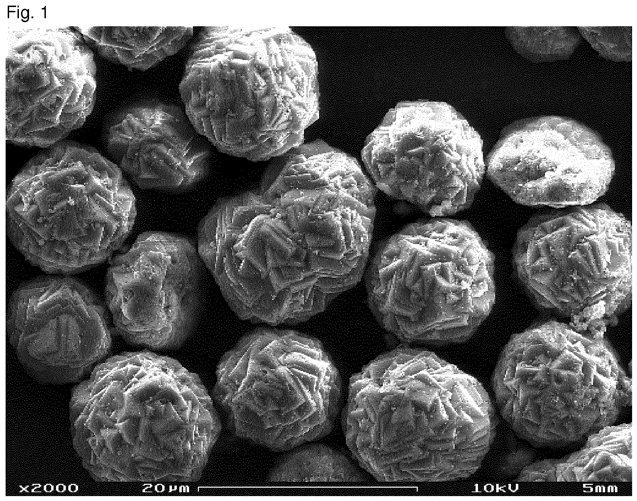

Die resultierenden kugelförmigen Calciumcarbonat-Teilchen wurden auf herkömmliche Weise abgetrennt und getrocknet. Sie wiesen einen mittleren Teilchendurchmesser von 12 µm auf. Eine typische REM-Aufnahme ist in Fig. 1 dargestellt.

### Beispiel 2 (Edukt für Verbundpulver gemäß der beanspruchten Erfindung)

500 ml VE-Wasser wurden in einem 1000 ml Becherglas vorgelegt. 125 g kugelförmige Calciumcarbonat-Teilchen nach Beispiel 1 wurden unter Rühren hinzugegeben und die resultierende Mischung wurde 5 min gerührt. 37,5 g einer 10%igen Natriummetaphosphat (NaPO₃)ₙ-Lösung wurden langsam zugegeben und die resultierende Mischung wurde 10 min gerührt. 75,0 g 10%iger Phosphorsäure wurden langsam zugegeben und die resultierende Mischung wurde 20 h gerührt. Der Niederschlag wird abgetrennt und über Nacht im Trockenschrank bei 130°C getrocknet. Die resultierenden kugelförmigen Calciumcarbonat-Teilchen wiesen ebenfalls einen mittleren Teilchendurchmesser von 12 µm auf.

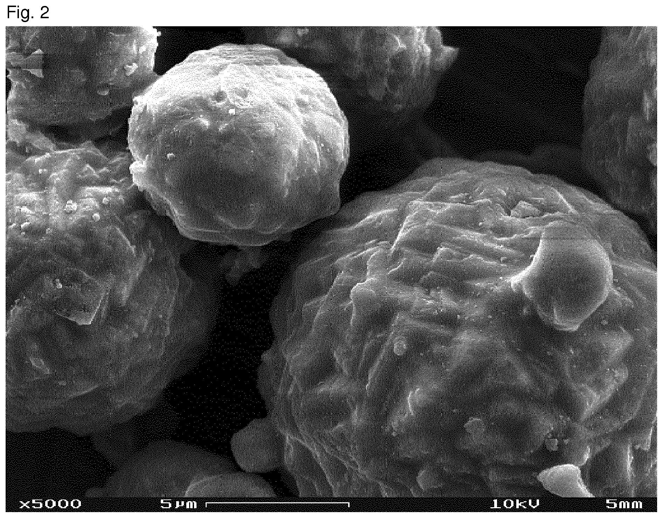

Eine REM-Aufnahme der kugelförmigen Calciumcarbonat-Teilchen ist in Fig. 2 dargestellt. Es ist auf der Oberfläche der kugelförmigen Calciumcarbonat-Teilchen eine dünne Phosphat-Schicht zu erkennen.

### Beispiel 3 (Verbundpulver gemäß der beanspruchten Erfindung)

Ein Verbundpulver aus kugelförmigen Calciumcarbonat-Teilchen und einem Polylactid (PLLA) wurde in Anlehnung an das in JP 62083029 A beschriebene Verfahren unter Verwendung des Geräts NHS-1 hergestellt. Es wurde mit 12°C kaltem Wasser gekühlt. Als Mutterpartikel wurden ein Polylactid-Granulat 1 und als die Babypartikel (Füllstoff) die kugelförmigen Calciumcarbonat-Teilchen aus Beispiel 1 verwendet.

39,5 g Polylactid-Granulat wurden mit 26,3 g CaCOs-Pulver vermengt und bei 6.400 U/min befüllt. Die Rotorgeschwindigkeit des Aggregats wurde auf 6.400 U/min (80 m/s) eingestellt und die zudosierten Materialien für 10 min verarbeitet. Die maximal erreichte Temperatur im Mahlraum des NHS-1 betrug 35°C. Es wurden insgesamt 7 Wiederholungen mit den gleichen Materialmengen und Maschineneinstellungen durchgeführt. Es wurden insgesamt 449 g Verbundpulver gewonnen. Das gewonnene Verbundpulver wurde durch ein 250 µm-Sieb manuell trocken gesiebt. Der Siebrückstand (Fraktion > 250 µm) betrug 0,4 %.

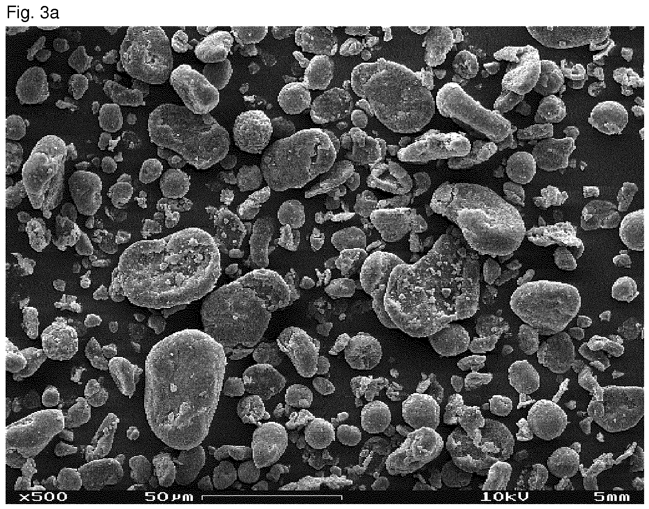

Eine REM-Aufnahme des erhaltenen Verbundpulvers ist in Fig. 3a dargestellt.

### Beispiele 4 bis 7 (Verbundpulver gemäß der beanspruchten Erfindung)

Es wurden weitere Verbundpulver analog Beispiel 3 hergestellt, wobei in Beispiel 5 die Kühlung bei ca. 20°C erfolgte. Es wurden jeweils 30 g Polylactid-Granulat mit 20 g CaCOs-Pulver vermengt. Die maximal erreichte Temperatur im Mahlraum des NHS-1 betrug für Beispiel 4 33°C, für Beispiel 5 58°C, für Beispiel 6 35°C und für Beispiel 7: 35°C. Die Produkte wurden gesiebt, um die Grobfraktion >250 µm nach Möglichkeit zu entfernen (manuelle Trocken-Siebung durch ein 250 µm-Sieb). In den Beispielen 4, 6 und 7 wurde zusätzlich die Fraktion < 20 µm nach Möglichkeit Stromklassiert (mittels Windsichten) oder durch Sieben (mittels einer Luftstrahlsiebmaschine) entfernt. Die eingesetzten Materialien, die Durchführung der Herstellung mit oder ohne Siebung/Windsichtung sowie die Eigenschaften der erhaltenen Verbundpulver werden in der folgenden Tabelle 3 zusammengefasst.

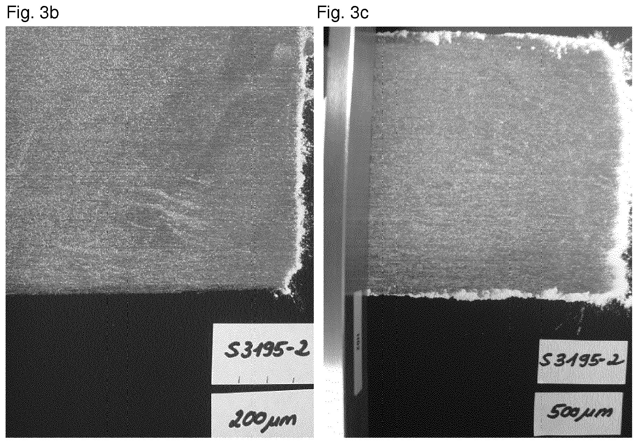

Fig. 3a, Fig. 3b und Fig. 3c zeigen eine REM-Aufnahme von Beispiel 3 sowie Aufnahmen von mehreren Rakelaufträgen (12,5 mm/s) von Beispiel 3 (Fig. 3b: 200 µm Rakel; Fig. 3c: 500 µm Rakel).

Die REM-Aufnahme des erhaltenen Verbundpulvers ist in der Fig. 3a dargestellt. Im Gegensatz zu der kantigen, unregelmäßigen Partikelform, die typisch für die kryogen gemahlenen Pulver sind, haben die Teilchen des gewonnenen Verbundpulvers eine für SLM-Verfahren sehr vorteilhafte runde Partikelform bzw. hohe Sphärizität. Die PLLA-Oberfläche ist spärlich mit kugelförmigen Calciumcarbonat-Teilchen und deren Fragmenten belegt. Die Probe hat eine breite Partikelgrößenverteilung mit erhöhtem Feinanteil.

Das Pulver ist eingeschränkt fließbar (Fig. 3b und 3c). Ein Pulverberg wird von der Rakel vor sich hergeschoben. Durch das eingeschränkte Fließverhalten, ausgelöst wahrscheinlich durch einen höheren Anteil an Feinpartikeln, entstehen mit beiden Rakeln nur sehr dünne Schichten.

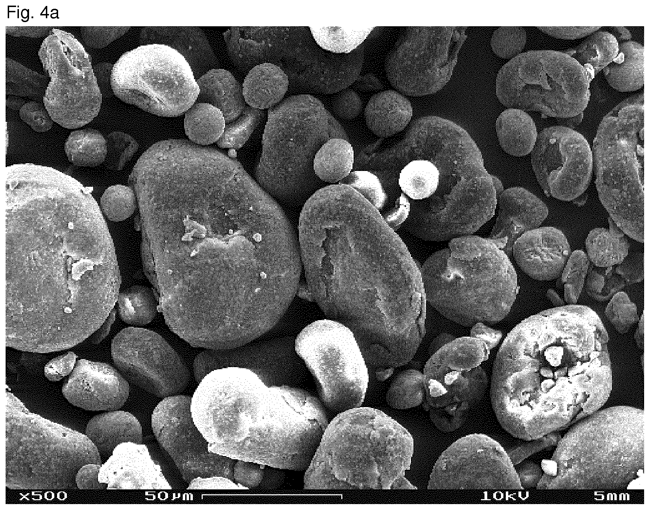

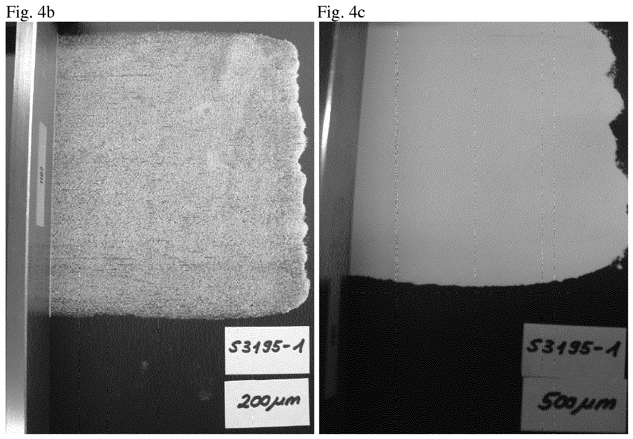

Fig. 4a, Fig. 4b und Fig. 4c zeigen eine REM-Aufnahme von Beispiel 4 sowie Aufnahmen von mehreren Rakelaufträgen (12,5 mm/s) von Beispiel 4 (Fig. 4b: 200 µm Rakel; Fig.4c: 500 µm Rakel).

Die REM-Aufnahme des erhaltenen Verbundpulvers ist in Fig. 4a dargestellt. Im Gegensatz zu der kantigen, unregelmäßigen Partikelform, die typisch für die kryogen gemahlenen Pulver sind, haben die Teilchen des gewonnenen Verbundpulvers eine für SLM-Verfahren sehr vorteilhafte runde Partikelform bzw. hohe Sphärizität. Die PLLA-Oberfläche ist spärlich mit kugelförmigen Calciumcarbonat-Teilchen und deren Fragmenten belegt. Die Probe hat deutlich schmalere Partikelgrößenverteilung mit wenig Feinanteil.

Das Pulver ist sehr gut fließbar und rakelbar (Fig. 4b und 4c). Auch die dünnen Schichten (200 µm) lassen sich aufrakeln und sind weitgehend frei von Rakelstreifen (Spurrillen). Die mit 500 µm gerakelte Pulverschicht ist homogen, dicht gepackt, glatt und frei von Rakelstreifen.

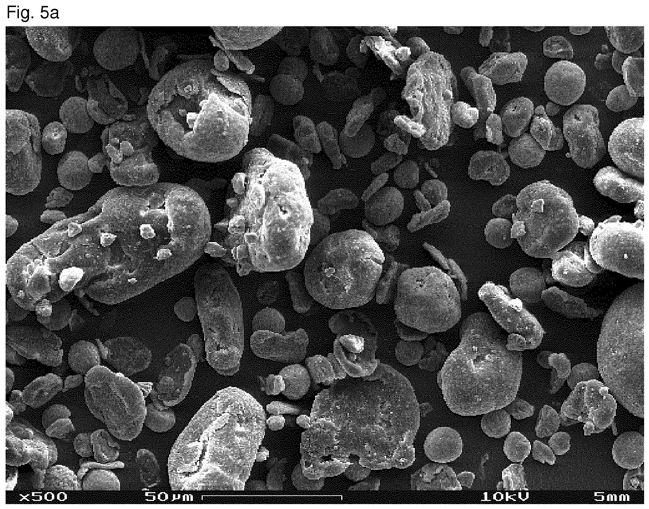

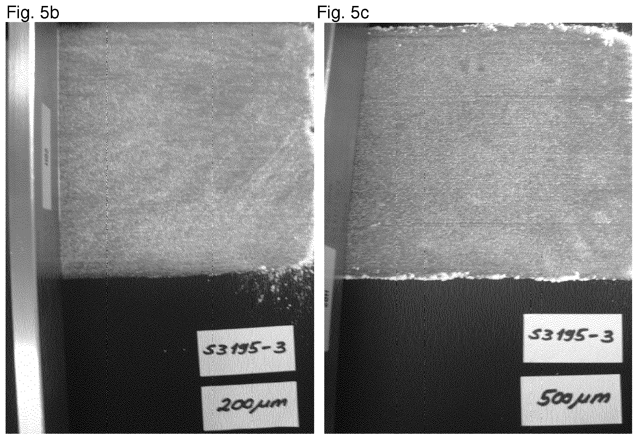

Fig. 5a, Fig. 5b und Fig. 5c zeigen eine REM-Aufnahme von Beispiel 5 sowie Aufnahmen von mehreren Rakelaufträgen (12,5 mm/s) von Beispiel 5 (Fig. 5b: 200 µm Rakel; Fig. 5c: 500 µm Rakel). Das Pulver ist eingeschränkt fließbar. Ein Pulverberg wird von der Rakel vor sich hergeschoben. Durch das eingeschränkte Fließverhalten, ausgelöst wahrscheinlich durch einen höheren Anteil an Feinpartikeln, entstehen mit beiden Rakeln nur sehr dünne Schichten.

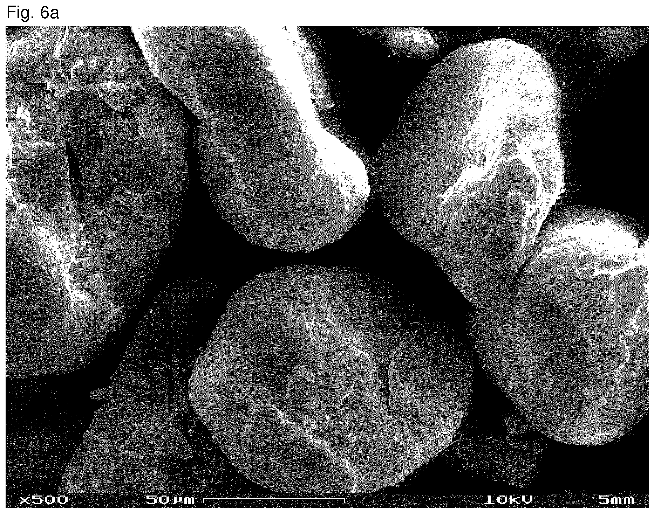

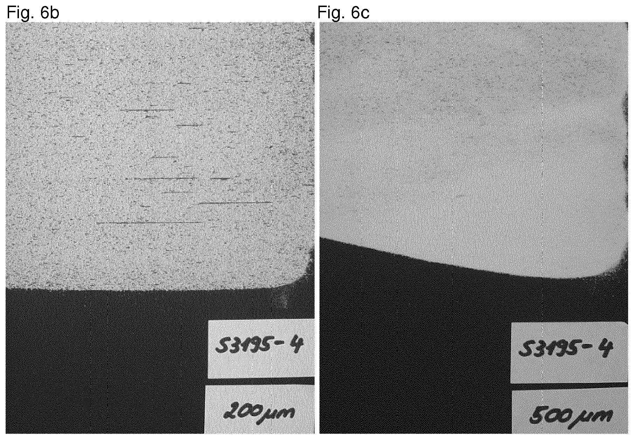

Fig. 6a, Fig. 6b und Fig. 6c zeigen eine REM-Aufnahme von Beispiel 6 sowie Aufnahmen von mehreren Rakelaufträgen (12,5 mm/s) von Beispiel 6 (Fig. 6b: 200 µm Rakel; Fig. 6c: 500 µm Rakel). Das Pulver ist gut fließbar und rakelbar. Auch die dünnen Schichten (200 µm) lassen sich aufrakeln. Einzelne Rakelstreifen durch vermutlich zu grobe Pulverpartikel sind erkennbar. Die mit 500 µm gerakelte Pulverschicht ist nicht ganz dicht gepackt, ist aber frei von Rakelstreifen.

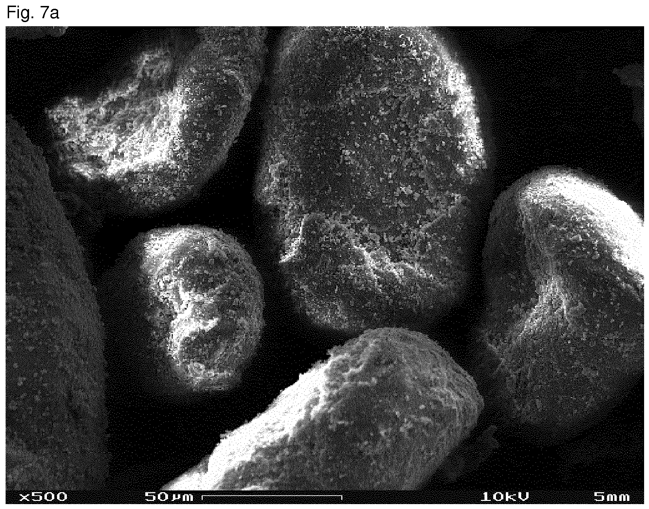

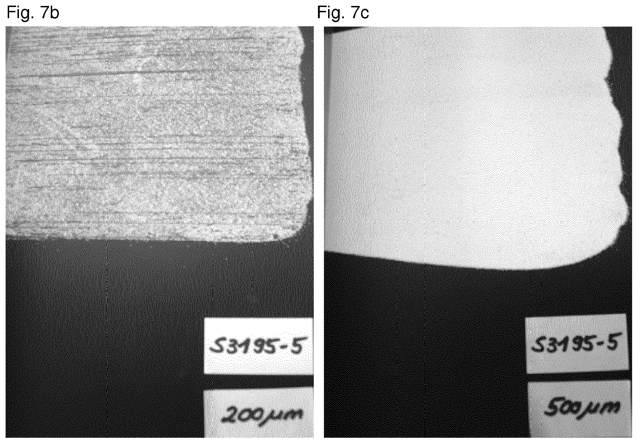

Fig. 7a, Fig. 7b und Fig. 7c zeigen eine REM-Aufnahme von Beispiel 7 sowie Aufnahmen von mehreren Rakelaufträgen (12,5 mm/s) von Beispiel 7 (Fig. 7b: 200 µm Rakel; Fig. 7c: 500 µm Rakel). Das Pulver ist fließbar und rakelbar. Auch die dünnen Schichten (200 µm) lassen sich aufrakeln. Sie sind nicht homogen und von Rakelstreifen vermehrt durchsetzt. Etwas eingeschränktes Fließverhalten ist wahrscheinlich durch zu grobe Pulverpartikel hervorgerufen. Die mit 500 µm gerakelte Pulverschicht ist homogen und frei von Rakelstreifen.

### Vergleich 1 (Vergleichsbeispiel)

Mikrostrukturierte Verbundteilchen aus kugelförmigen Calciumcarbonat-Teilchen aus Beispiel 1 und einem amorphen Polylactid (PDLLA) wurden in Anlehnung an das in JP 62083029 A beschriebene Verfahren unter Verwendung des Geräts NHS-1 hergestellt. Es wurde mit 12°C kaltem Wasser gekühlt. Als Mutterpartikel wurde ein Polylactid-Granulat 3 und als die Babypartikel die kugelförmigen Calciumcarbonat-Teilchen aus Beispiel 1 verwendet.

39,5 g Polylactid-Granulat wurden mit 10,5 g CaCOs-Pulver vermengt und bei 8.000 U/min befüllt. Die Rotorgeschwindigkeit des Aggregats wurde auf 8.000 U/min (100 m/s) eingestellt und die zudosierten Materialien für 1,5 min verarbeitet. Die maximal erreichte Temperatur im Mahlraum des NHS-1 betrug 71 °C. Es wurden insgesamt 49 Wiederholungen mit den gleichen Materialmengen und Maschineneinstellungen durchgeführt. Es wurden insgesamt 2376 g strukturierte Verbundteilchen gewonnen. Die gewonnenen strukturierten Verbundteilchen wurden für die Messung der Teilchengrößenverteilung durch ein 800 µm-Sieb manuell trocken gesiebt. Der Siebrückstand (Fraktion > 800 µm) betrug 47 %.

Die Eigenschaften der erhaltenen mikrostrukturierten Verbundteilchen werden in der folgenden Tabelle 3 zusammengefasst.

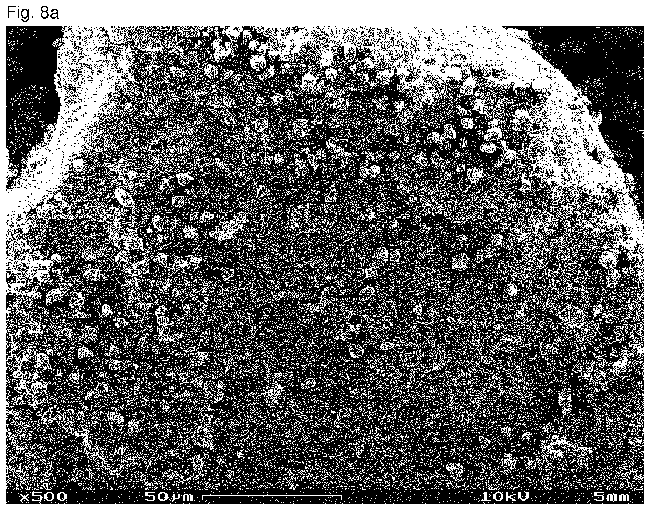

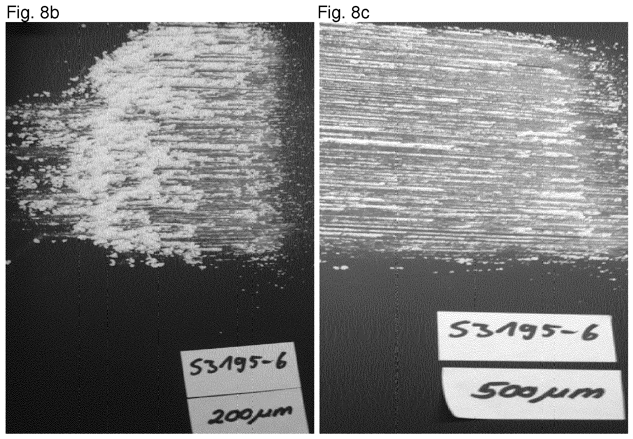

Fig. 8a, Fig. 8b und Fig. 8c zeigen eine REM-Aufnahme von Vergleich 1 sowie Aufnahmen von mehreren Rakelaufträgen (12,5 mm/s) von Vergleich 1 (Fig. 8b: 200 µm Rakel; Fig. 8c: 500 µm Rakel). Das Pulver ist schlecht fließbar und kann nicht zu 200 bzw. 500 µm dünnen Schichtstärken gerakelt werden. Die zu groben, irregulären Partikel verklemmen sich beim Aufrakeln. Es entstehen inhomogene Schichten mit sehr häufigen und ausgeprägten Rakelstreifen.

Die REM-Analyse zeigt, dass die Oberflächen der strukturierten Verbundpulver spärlich mit kugelförmigen Calciumcarbonat-Teilchen und deren Fragmenten belegt sind. Im Vergleich zu den Beispielen 3 - 7 haben die Partikel eine unregelmäßigere Partikelgeometrie.

### Beispiel 8 (Verbundpulver nicht gemäß der beanspruchten Erfindung)

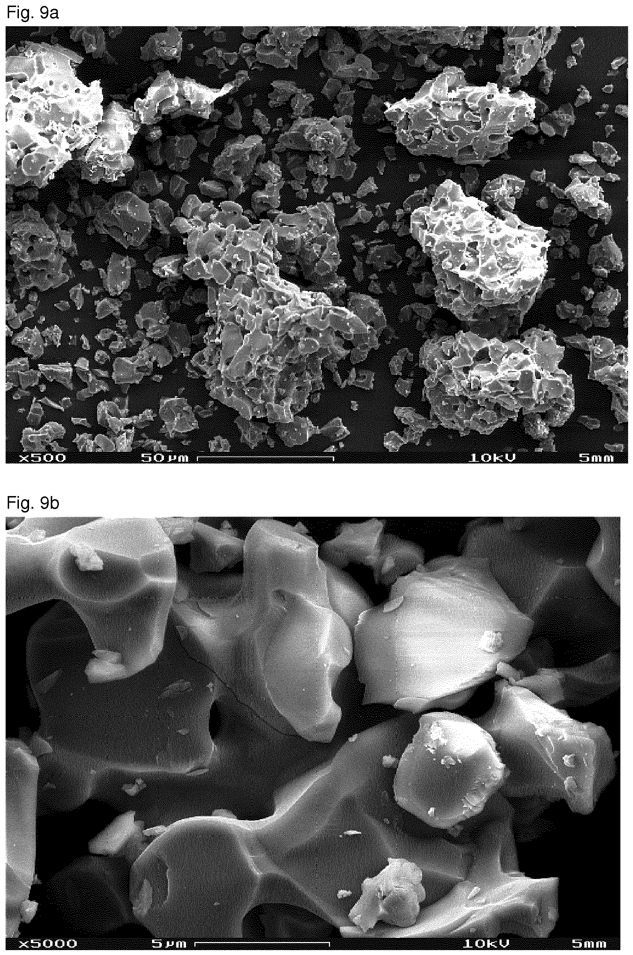

Ein Verbundpulver aus β-Tricalciumphosphat -Teilchen und einem Polylactid (PDLLA) wurde in Anlehnung an das in JP 62083029 A beschriebene Verfahren unter Verwendung des Geräts NHS-1 hergestellt. Es wurde mit 12°C kaltem Wasser gekühlt. Als Mutterpartikel wurde ein Polylactid-Granulat 3 und als die Babypartikel β-Tricalciumphosphat (ß-TCP; d₂₀=30 µm; d₅₀=141 µm; d₉₀=544 µm) verwendet. Die REM-Aufnahme des verwendeten ß-TCP's werden in Fig. 9a und Fig. 9b gezeigt.

30,0 g Polylactid-Granulat wurden mit 20,0 g β-TCP-Pulver vermengt und bei 6.400 U/min befüllt. Die Rotorgeschwindigkeit des Aggregats wurde auf 6.400 U/min (80 m/s) eingestellt und die zudosierten Materialien für 10 min verarbeitet.

Es wurden insgesamt 5 Wiederholungen mit den gleichen Materialmengen und Maschineneinstellungen durchgeführt. Es wurden insgesamt 249 g Verbundpulver gewonnen. Das Produkt wurden gesiebt, um die Grobfraktion >250 µm nach Möglichkeit zu entfernen (manuelle Trocken-Siebung durch ein 250 µm-Sieb). Anschließend wurde der Feinanteil < 20 µm über ein 20 µm-Sieb mittels einer Luftstrahlsiebmaschine abgetrennt.

### Beispiel 9 (Verbundpulver nicht gemäß der beanspruchten Erfindung)

Ein Verbundpulver aus rhomboedrischen Calciumcarbonat-Teilchen und einem Polylactid (PDLLA) wurde in Anlehnung an das in JP 62083029 A beschriebene Verfahren unter Verwendung des Geräts NHS-1 hergestellt. Es wurde mit 12°C kaltem Wasser gekühlt. Als Mutterpartikel wurde ein Polylactid-Granulat 3 und als die Babypartikel rhomboedrische Calciumcarbonat-Teilchen (d₂₀=11 µm; d₅₀=16 µm; d₉₀=32 µm) verwendet.

30,0 g Polylactid-Granulat wurden mit 20,0 g der rhomboedrischen Calciumcarbonat-Teilchen vermengt und bei 6.400 U/min befüllt. Die Rotorgeschwindigkeit des Aggregats wurde auf 6.400 U/min (80 m/s) eingestellt und die zudosierten Materialien für 10 min verarbeitet. Es wurden insgesamt 5 Wiederholungen mit den gleichen Materialmengen und Maschineneinstellungen durchgeführt. Es wurden insgesamt 232 g Verbundpulver gewonnen. Das Produkt wurden gesiebt, um die Grobfraktion >250 µm nach Möglichkeit zu entfernen (manuelle Trocken-Siebung durch ein 250 µm-Sieb). Anschließend wurde der Feinanteil < 20 µm über ein 20 µm-Sieb mittels einer Luftstrahlsiebmaschine abgetrennt.

### Beispiel 10 (Verbundpulver nicht gemäß der beanspruchten Erfindung)

Ein Verbundpulver aus gemahlenen Calciumcarbonat-Teilchen und einem Polylactid (PDLLA) wurde in Anlehnung an das in JP 62083029 A beschriebene Verfahren unter Verwendung des Geräts NHS-1 hergestellt. Es wurde mit 12°C kaltem Wasser gekühlt. Als Mutterpartikel wurden ein Polylactid-Granulat 3 und als die Babypartikel gemahlenes Calciumcarbonat (GCC; d₂₀=15 µm; d₅₀=46 µm; d₉₀=146 µm) verwendet.

30,0 g Polylactid-Granulat wurden mit 20,0 g GCC vermengt und bei 6.400 U/min befüllt. Die Rotorgeschwindigkeit des Aggregats wurde auf 6.400 U/min (80 m/s) eingestellt und die zudosierten Materialien für 10 min verarbeitet. Es wurden insgesamt 5 Wiederholungen mit den gleichen Materialmengen und Maschineneinstellungen durchgeführt. Es wurden insgesamt 247 g Verbundpulver gewonnen. Das Produkt wurden gesiebt, um die Grobfraktion >250 µm nach Möglichkeit zu entfernen (manuelle Trocken-Siebung durch ein 250 µm-Sieb). Anschließend wurde der Feinanteil < 20 µm über ein 20 µm-Sieb mittels einer Luftstrahlsiebmaschine abgetrennt.

**Tabelle 3**

| | Beispiel 3 | Beispiel 4 | Beispiel 5 | Beispiel 6 | Beispiel 7 | Vergleich 1 |
|---|---|---|---|---|---|---|
| Zusammensetzung für die Herstellung des Verbundpulvers mit mikrostrukturierten Teilchen | | | | | | |
| m(Beispiel 1) [Gew.-%] | 40 | 40 | 0 | 40 | 40 | 20 |
| m(Beispiel 2) [Gew.-%] | 0 | 0 | 40 | 0 | 0 | 0 |

| Polylactid | Granulat 1 | Granulat 1 | Granulat 1 | Granulat 2 | Granulat 3 | Granulat 3 |
|---|---|---|---|---|---|---|
| m(Polylactid) [Gew.-%] | 60 | 60 | 60 | 60 | 60 | 80 |

| Herstellung des Verbundpulvers mit mikrostrukturierten Teilchen | | | | | | |
|---|---|---|---|---|---|---|
| Siebung | < 250 µm | < 250 µm | < 250 µm | < 250 µm | < 250 µm | < 800 µm |
| | | < 20 µm (Windsichtung) | | < 20 µm (Luftstrahlsiebung) | < 20 µm (Luftstrahlsiebung) | (nur für Messung der Teilchengrößenverteilung) |
| | | | | | | |
| CaCOs-Gehalt [Gew.-%]¹ | 41,0 | 22,4 | 35,0 | 19,5 | 22,3 | 22,4 (Mittelwert aus 5 Messungen) |
| T_{P} [°C]¹ | 291 | 310 | 341 | 304 | 286 | 319 (Mittelwert aus 5 Messungen) |
| d₅₀ [µm]¹ | 25 | 47 | 26 | 112 | 136 | 228 |
| Anteil <20 µm [Vol.-%]¹ | 43,6 | 13,7 | 37,7 | 0,3 | 2,3 | 20,6 |
| d₂₀ [µm]¹ | 9 | 26 | 14 | 69 | 80 | |
| d₉₀ [µm]¹ | 86 | 102 | 70 | 223 | 247 | |
| d₂₀/d₅₀ [%] | 36 | 52 | 54 | 62 | 59 | |
| Feuchte [Gew.-%]¹ | 0,8 | 0,6 | 0,5 | 0,9 | 0,9 | 0,3 |
| Inhärente Viskosität [dl/g] | 1,0 | 1,0 | 0,9 | 1,9 | 1,9 | 1,9 |
| Drei-Punkt-Biegefestigkeit [MPa] | 66 | 68 | 77 | 84 | 67 | 79 |
| E-Modul [N/mm²] | 4782 | 3901 | 4518 | 3530 | 3594 | 3420 |
| Fließfähigkeit | 4 | 1 | 4 | 2 | 3 | 5 |
| Zytotoxitätstest | nicht zytotoxisch | nicht zytotoxisch | nicht zytotoxisch | - | nicht zytotoxisch | nicht zytotoxisch |

| | | | | | | |
|---|---|---|---|---|---|---|
| ¹: mindestens Zweifachbestimmung | | | | | | |

**Tabelle 3 (Fortsetzung)**

| | Beispiel 8 | Beispiel 9 | Beispiel 10 |
|---|---|---|---|
| Zusammensetzung für die Herstellung des Verbundpulvers mit mikrostrukturierten Teilchen | | | |
| m(Füllstoff) [Gew.-%] | 40 | 40 | 40 |

| Polylactid | Granulat 3 | Granulat 3 | Granulat 3 |
|---|---|---|---|
| m(Polylactid) [Gew.-%] | 60 | 60 | 60 |

| Herstellung des Verbundpulvers mit mikrostrukturierten Teilchen | | | |
|---|---|---|---|
| Siebung | < 250 µm | < 250 µm | < 250 µm |
| | < 20 µm Luftstrahlsiebung | < 20 µm Luftstrahlsiebung | < 20 µm Luftstrahlsiebung |
| | | | |
| Füllstoff-Gehalt [Gew.-%]* | 24,9 | 24,2 | 26,6 |
| T_{P} [°C] | 341°C | 303°C | 303°C |
| d₂₀ [µm] | 85 | 74 | 75 |
| d₅₀ [µm] | 131 | 128 | 120 |
| d₉₀ [µm] | 226 | 257 | 230 |
| Anteil <20 µm [Vol.-%] | 3,0 | 4,5 | 1,6 |
| Feuchte [Gew.-%] | 0,6 | 0,6 | 0,6 |
| Inhärente Viskosität [dl/g] | 1,8 | 1,8 | 1,9 |

Vergleich 2, Beispiel 11 (Verbundpulver gemäß der beanspruchten Erfindung), Beispiel 12 (Verbundpulver gemäß der beanspruchten Erfindung), Beispiel 13 (Verbundpulver gemäß der beanspruchten Erfindung), Beispiel 14 (Verbundpulver gemäß der beanspruchten Erfindung) und Beispiel 15

PLA-Pellets wurden als reine Pellets sowie mit 4 verschiedenen Füllstoffen (25 Gew.-%) mit einem Brabender-Plasti-Corder gemischt und aufgeschmolzen. Die Temperatur der Kammer betrug 190°C bei einer Drehzahl von 50 U/min. Pellets und Füllstoffpulver wurden für 5 Minuten gemischt, anschließend wurden ca. 16 g der Mischung in der hydraulischen Presse für 5 Minuten bei einem Druck von 0,96 - 1,2 MPa gepresst.

Als Polymer wurde in allen Beispielen PLA (NatureWorks Ingeo TM Biopolymer 3251D) verwendet. In Vergleich 2 wurden keine Calciumcarbonat-Teilchen zugesetzt. In Beispiel 11 wurden Calciumcarbonat-Teilchen gemäß Beispiel 1 zugesetzt. In Beispiel 12 wurden Calciumcarbonat-Teilchen gemäß Beispiel 2 zugesetzt. In Beispiel 13 wurden Calciumcarbonat-Teilchen zugesetzt, wobei die Herstellung der Teilchen analog Beispiel 2 allerdings ohne Zugabe von Phosphorsäure erfolgte. In Beispiel 14 wurden Calciumcarbonat-Teilchen zugesetzt, wobei die Herstellung der Teilchen analog Beispiel 2 allerdings ohne Zugabe von Natriummetaphosphat (NaPO₃)ₙ) erfolgte. In Beispiel 15 (nicht erfindungsgemäß) wurden mit Stearinsäure-beschichtete Calciumcarbonat-Teilchen zugesetzt, die auf herkömmliche Art und Weise erhalten wurden.

Charakterisierung der PLA-Komposite von Vergleich 2 und Beispiel 11-15
a) Mechanische Eigenschaften
   Die mechanischen Eigenschaften von PLA und der Komposite wurden mit der Universalprüfmaschine UTM 1445 von Zwick/Roell getestet. Die Zugfestigkeit, das Elastizitätsmodul und die Dehnung der Materialien wurden hierbei bestimmt. Die Prüfgeschwindigkeit betrug 10 mm/ min bei einer Messlänge von 50 mm.
b) Thermische Eigenschaften
   Die thermische Stabilität der Proben wurde mittels Thermogravimetrie bestimmt. Die thermogravimetrischen Messungen wurden mit einem STA 6000 der Firma Perkin Elmer unter Stickstoff im Bereich von 40°C bis 1000°C bei einer Heizrate von 20°C/min durchgeführt.
c) Optische Bewertung der Proben (**Noten von 1-3)
   1 = transparentes reines Polymer; keine Verfärbung durch den thermischen Abbau erkennbar
   2 = weißes Polymercompound; Farbwechsel zu weiß durch Zugabe des Füllstoffes; keine Verfärbung durch den thermischen Abbau erkennbar
   3 = Braune Färbung durch thermischen Abbau des Compounds

Die Zugabe der CaCOs-Teilchen in die PLA-Matrix führte zu einem Farbwechsel von transparentem reinen PLA zu weißen Verbundwerkstoffen bei allen Füllstoffe außer Beispiel 15. Bei der Probe mit Stearinsäure-beschichteten Calciumcarbonat-Teilchen veränderte sich die Farbe zu einem Hellbraun, was auf eine Degradation des Polymers hinweist. Alle anderen Proben zeigen keinerlei Degradationserscheinungen.

Die beobachteten Eigenschaften werden in Tabelle 4 zusammengefasst.

**Tabelle 4**

| | Vergleich 2 | Beispiel 11 | Beispiel 12 | Beispiel 13 | Beispiel 14 | Beispiel 15 |
|---|---|---|---|---|---|---|
| CaCOs-Teilchen | | Beispiel 1 | Beispiel 2 | Beispiel 2 ohne Zugabe von Phosphorsäure | Beispiel 2 ohne Zugabe von Natriummetaphosphat | mit Stearinsäure beschichtet (1,0 %) |
| pH¹⁾ (sofort / 24 h) | | 10,0 / 10,0 | 6,1 / 6,2 | 8,9 / 9,0 | 7,0 / 7,0 | - |
| Feuchte [%] | | 0,1 | 0,1 | 0,1 | 0,1 | 0,1 |
| d₅₀ [µm] | | 12,1 | 12,2 | 12,0 | 14,3 | 14,2 |
| Spez. Oberfläche [m²/g] | | 1,1 | 0,2 | 0,6 | 0,9 | 4,9 |
| P₂O₅-Gehalt [%] | | 0,3 | 3,1 | 0,4 | 6,8 | - |

| Qualitative Phasenanalyse | | Calcit | Calcit | Calcit | Calcit + Brushit | |
|---|---|---|---|---|---|---|
| Zugfestigkeit [MPa] | 47,99 | 44,57 | 40,56 | 40,20 | 37,95 | 41,39 |
| Elastizitätsmodul [MPa] | 1345,0 | 1680,4 | 1718,9 | 1601,9 | 1625,8 | 1627,1 |
| Onset-Temperatur TGA) [°C] | 348,8 | 326,1 | 360,3 | 337,4 | 358,4 | 322,9 |
| Peak-Temperatur (TGA) [°C] | 377,6 | 356,5 | 380,3 | 368,1 | 380,8 | 354,5 |
| Benotung der Prüfkörper** | 1 | 2 | 2 | 2 | 2 | 3 |

## Patentansprüche

1. Verbundpulver mit mikrostrukturierten Teilchen, erhältlich durch ein Verfahren, bei welchem man große Teilchen mit kleinen Teilchen verbindet, wobei
- die großen Teilchen einen mittleren Teilchendurchmesser im Bereich von 0,1 µm bis 10 mm aufweisen,
- die großen Teilchen mindestens ein Polymer umfassen,
- die kleinen Teilchen auf der Oberfläche der großen Teilchen angeordnet und/oder inhomogen innerhalb der großen Teilchen verteilt sind,
- die kleinen Teilchen kugelförmige gefällte Calciumcarbonat-Teilchen mit einem mittleren Durchmesser im Bereich von 0,05 µm bis 50,0 µm umfassen,
**dadurch gekennzeichnet, dass**
die kugelförmigen Calciumcarbonat-Teilchen nach einem Verfahren erhältlich sind, bei welchem man
a. eine Calciumhydroxid-Suspension vorlegt,
b. in die Suspension aus Schritt a. Kohlendioxid oder eine Kohlendioxid-haltige Gasmischung einleitet und
c. entstehende Calciumcarbonat-Teilchen abtrennt,
wobei man weiterhin 0,3 Gew.-% bis 0,7 Gew.-% mindestens einer Aminotrialkylenphosphonsäure zugibt, und
die Teilchen des Verbundpulvers eine mittlere Teilchengröße d₅₀ im Bereich von 10 µm bis kleiner 200 µm aufweisen.

2. Verbundpulver nach Anspruch 1, umfassend Calciumcarbonat-Teilchen, erhältlich nach einem Verfahren, bei welchem man Aminotrimethylenphosphonsäure, Aminotriethylenphosphonsäure, Aminotripropylenphosphonsäure und/oder Aminotributylenphosphonsäure zugibt.

3. Verbundpulver nach Anspruch 1 oder 2, umfassend Calciumcarbonat-Teilchen, erhältlich nach einem Verfahren, bei welchem man die Einleitung des Kohlendioxids oder der Kohlendioxid-haltigen Gasmischung solange durchführt, bis die Reaktionsmischung einen pH-Wert kleiner 9 aufweist.

4. Verbundpulver nach mindestens einem der vorangehenden Ansprüche, umfassend Calciumcarbonat-Teilchen, erhältlich nach einem Verfahren, bei welchem man die Umsetzung der Calciumhydroxid-Suspension mit dem Kohlendioxid oder der Kohlendioxid-haltigen Gasmischung bei einer Temperatur kleiner 25°C durchführt.

5. Verbundpulver nach mindestens einem der vorangehenden Ansprüche, umfassend Calciumcarbonat-Teilchen, erhältlich nach einem Verfahren, bei welchem man Kohlendioxid oder eine Kohlendioxid-haltige Gasmischung mit einer Gasdurchflußmenge im Bereich von 0,02 l CO₂ / (h*g Ca(OH)₂) bis 2,0 l CO₂ / (h*g Ca(OH)₂) in die Calciumhydroxid-Suspension leitet.

6. Verbundpulver nach mindestens einem der vorangehenden Ansprüche, **dadurch gekennzeichnet, dass** die kugelförmigen Calciumcarbonat-Teilchen einen mittleren Durchmesser kleiner 30,0 µm, insbesondere kleiner 20,0 µm, aufweisen.

7. Verbundpulver nach mindestens einem der vorangehenden Ansprüche, **dadurch gekennzeichnet, dass** die kugelförmigen Calciumcarbonat-Teilchen eine Größenverteilung haben, bei welcher mindestens 90,0 Gew.-% aller Calciumcarbonat-Teilchen einen Teilchendurchmesser im Bereich von mittlerer Teilchendurchmesser -30% bis mittlerer Teilchendurchmesser +30% aufweisen.

8. Verbundpulver nach mindestens einem der vorangehenden Ansprüche, **dadurch gekennzeichnet, dass** die kugelförmigen Calciumcarbonat-Teilchen einen Formfaktor, definiert als der Quotient aus minimalem Teilchendurchmesser und maximalem Teilchendurchmesser, größer 0,90 aufweisen.

9. Verbundpulver nach mindestens einem der vorangehenden Ansprüche, **dadurch gekennzeichnet, dass** die großen Teilchen mindestens ein thermoplastisches Polymer umfassen.

10. Verbundpulver nach mindestens einem der vorangehenden Ansprüche, **dadurch gekennzeichnet, dass** die großen Teilchen mindestens ein resorbierbares Polymer umfassen.

11. Verbundpulver nach Anspruch 10, **dadurch gekennzeichnet, dass** das resorbierbare Polymer eine inhärente Viskosität, gemessen in Chloroform bei 25°C, 0,1 % Polymerkonzentration, im Bereich von 0,3 dl/g bis 8,0 dl/g aufweist.

12. Verbundpulver nach mindestens einem der vorangehenden Ansprüche, **dadurch gekennzeichnet, dass** die großen Teilchen Poly-D-, Poly-L- und/oder Poly-D,L-milchsäure umfassen.

13. Verbundpulver nach mindestens einem der vorangehenden Ansprüche, **dadurch gekennzeichnet, dass** die großen Teilchen mindestens einen resorbierbaren Polyester mit einem Zahlenmittel des Molekulargewichts im Bereich von 500 g/mol bis 1.000.000 g/mol umfassen.

14. Verbundpulver nach mindestens einem der vorangehenden Ansprüche, **dadurch gekennzeichnet, dass** die großen Teilchen mindestens ein Polyamid umfassen.

15. Verbundpulver nach mindestens einem der vorangehenden Ansprüche, **dadurch gekennzeichnet, dass** die großen Teilchen mindestens ein Polyurethan umfassen.

16. Verbundpulver nach mindestens einem der vorangehenden Ansprüche, **dadurch gekennzeichnet, dass** der Gewichtsanteil der gefällten Calciumcarbonat-Teilchen, bezogen auf das Gesamtgewicht des Verbundpulvers, mindestens 0,1 Gew.-% beträgt.

17. Verbundpulver nach mindestens einem der vorangehenden Ansprüche, **dadurch gekennzeichnet, dass** das Verbundpulver, bezogen auf das Gesamtgewicht des Verbundpulvers, 40,0 Gew.-% bis 80,0 Gew.-% PLLA und 20,0 Gew.-% bis 60,0 Gew.-% gefällte Calciumcarbonat-Teilchen umfassen.

18. Verwendung eines Verbundpulvers nach mindestens einem der vorangehenden Ansprüche als Additiv, insbesondere als Polymeradditiv, als Zusatzsatzstoff oder Ausgangsmaterial für Compoundierung, für die Herstellung von Bauteilen, für Anwendungen in der Medizintechnik und/oder in der Mikrotechnik und/oder für die Herstellung von geschäumten Gegenständen.

19. Bauteil, erhältlich durch selektives Lasersintern einer Zusammensetzung, umfassend ein Verbundpulver nach mindestens einem der Ansprüche 1 bis 17, ausgenommen Implantate für Anwendungen im Bereich der Neuro-, Mund-, Kiefer-, Gesichts-, Hals-, Nasen- und Ohrenchirurgie sowie der Hand-, Fuß-, Thorax-, Rippen- und Schulterchirurgie.

20. Kugelförmige Calciumcarbonat-Teilchen, erhältlich nach einem Verfahren, bei welchem man
a. eine Calciumhydroxid-Suspension vorlegt,
b. in die Suspension aus Schritt a. Kohlendioxid oder eine Kohlendioxid-haltige Gasmischung einleitet und
c. entstehende Calciumcarbonat-Teilchen abtrennt,
wobei man weiterhin 0,3 Gew.-% bis 0,7 Gew.-% mindestens einer Aminotrialkylenphosphonsäure zugibt.

21. Verwendung der kugelförmigen Calciumcarbonat-Teilchen nach Anspruch 20 als Additiv für Papier, Kunststoffe, Farben und/oder Lacke, Elastomere sowie Kleb- und Dichtstoffe, in der Bauchemie, in Trockenmörtel und in der Medizintechnik, insbesondere als Additiv in resorbierbaren Polymeren.

## Claims

1. A composite powder containing microstructured particles obtainable by means of a method in which large particles are combined with small particles, wherein
- the large particles have an average particle diameter within the range from 0.1 µm to 10 mm,
- the large particles comprise at least one polymer,
- the small particles are arranged on the surface of the large particles and/or distributed inhomogeneously within the large particles,
- the small particles comprise sphere-shaped precipitated calcium carbonate particles having an average diameter within the range from 0.05 µm to 50.0 µm,
**characterized in that**
the sphere-shaped calcium carbonate particles are obtainable by means of a method in which
a. a calcium hydroxide suspension is initially charged,
b. carbon dioxide or a carbon dioxide-containing gas mixture is introduced into the suspension from step a. and
c. resultant calcium carbonate particles are separated off,
with 0.3% by weight to 0.7% by weight of at least one aminotri-salkylenephosphonic acid being further added, and
the particles of the composite powder have an average particle size d₅₀ within the range from 10 µm to less than 200 µm.

2. The composite powder as claimed in claim 1, comprising calcium carbonate particles obtainable by means of a method in which aminotrismeth-ylenephosphonic acid, aminotrisethylenephosphonic acid, aminotrispropyl-enephosphonic acid and/or aminotrisbutylenephosphonic acid are added.

3. The composite powder as claimed in claim 1 or 2, comprising calcium carbonate particles obtainable by means of a method in which the carbon dioxide or the carbon dioxide-containing gas mixture is introduced until the reaction mixture has a pH less than 9.

4. The composite powder as claimed in at least one of the preceding claims, comprising calcium carbonate particles obtainable by means of a method in which the conversion of the calcium hydroxide suspension with the carbon dioxide or the carbon dioxide-containing gas mixture is carried out at a temperature less than 25°C.

5. The composite powder as claimed in at least one of the preceding claims, comprising calcium carbonate particles obtainable by means of a method in which carbon dioxide or a carbon dioxide-containing gas mixture is introduced into the calcium hydroxide suspension at a gas flow rate within the range from 0.02 L CO₂ / (h*g Ca(OH)₂) to 2.0 L CO₂ / (h*g Ca(OH)₂).

6. The composite powder as claimed in at least one of the preceding claims, **characterized in that** the sphere-shaped calcium carbonate particles have an average diameter less than 30.0 µm, especially less than 20.0 µm.

7. The composite powder as claimed in at least one of the preceding claims, **characterized in that** the sphere-shaped calcium carbonate particles have a size distribution in which at least 90.0% by weight of all calcium carbonate particles have a particle diameter within the range of average particle diameter - 30% to average particle diameter +30%.

8. The composite powder as claimed in at least one of the preceding claims, **characterized in that** the sphere-shaped calcium carbonate particles have a shape factor, defined as the quotient formed from minimum particle diameter and maximum particle diameter, greater than 0.90.

9. The composite powder as claimed in at least one of the preceding claims, **characterized in that** the large particles comprise at least one thermoplastic polymer.

10. The composite powder as claimed in at least one of the preceding claims, **characterized in that** the large particles comprise at least one resorbable polymer.

11. The composite powder as claimed in claim 10, **characterized in that** the resorbable polymer has an inherent viscosity, measured in chloroform at 25°C and 0.1% polymer concentration, within the range from 0.3 dL/g to 8.0 dL/g.

12. The composite powder as claimed in at least one of the preceding claims, **characterized in that** the large particles comprise poly-D-, poly-L- and/or poly-D,L-lactic acid.

13. The composite powder as claimed in at least one of the preceding claims, **characterized in that** the large particles comprise at least one resorbable polyester having a number-average molecular weight within the range from 500 g/mol to 1 000 000 g/mol.

14. The composite powder as claimed in at least one of the preceding claims, **characterized in that** the large particles comprise at least one polyamide.

15. The composite powder as claimed in at least one of the preceding claims, **characterized in that** the large particles comprise at least one polyurethane.

16. The composite powder as claimed in at least one of the preceding claims, **characterized in that** the proportion by weight of the precipitated calcium carbonate particles, based on the total weight of the composite powder, is at least 0.1% by weight.

17. The composite powder as claimed in at least one of the preceding claims, **characterized in that** the composite powder comprise, based on the total weight of the composite powder, 40.0% by weight to 80.0% by weight of PLLA and 20.0% by weight to 60.0% by weight of precipitated calcium carbonate particles.

18. The use of a composite powder as claimed in at least one of the preceding claims as additive, especially as polymer additive, as additive substance or starting material for compounding, for the production of components, for applications in medical technology and/or in microtechnology and/or for the production of foamed articles.

19. A component obtainable by selective laser sintering of a composition comprising a composite powder as claimed in at least one of claims 1 to 17, except for implants for uses in the field of neurosurgery, oral surgery, jaw surgery, facial surgery, neck surgery, nose surgery and ear surgery as well as hand surgery, foot surgery, thorax surgery, rib surgery and shoulder surgery.

20. Sphere-shaped calcium carbonate particles obtainable by means of a method in which
a. a calcium hydroxide suspension is initially charged,
b. carbon dioxide or a carbon dioxide-containing gas mixture is introduced into the suspension from step a. and
c. resultant calcium carbonate particles are separated off,
with 0.3% by weight to 0.7% by weight of at least one aminotri-salkylenephosphonic acid being further added.

21. The use of the sphere-shaped calcium carbonate particles as claimed in claim 20 as additive for paper, plastics, paints and/or varnishes, elastomers as well as adhesives and sealants, in construction chemistry, in dry mortar and in medical technology, especially as additive in resorbable polymers.

## Revendications

1. Poudre composite à particules microstructurées, susceptible d'être obtenue par un procédé consistant à lier des particules de grande taille avec des particules de petite taille,
- les particules de grande taille présentant un diamètre moyen de particule de l'ordre de 0,1 µm à 10 mm,
- les particules de grande taille comprenant au moins un polymère,
- les particules de petite taille étant disposées sur la surface des particules de grande taille et / ou distribuées de manière non homogène à l'intérieur des particules de grande taille,
- les particules de petite taille comprenant des particules de carbonate de calcium précipitées de forme sphérique, d'un diamètre moyen de l'ordre de 0,05 µm à 50,0 µm,
**caractérisée en ce que**
les particules de carbonate de calcium de forme sphérique sont susceptibles d'être obtenues selon un procédé consistant à
a. prendre une suspension d'hydroxyde de calcium,
b. introduire dans la suspension de l'étape a. du dioxyde de carbone ou un mélange gazeux contenant du dioxyde de carbone et
c. séparer les particules de carbonate de calcium obtenues,
une quantité de 0,3 % en poids à 0,7 % en poids d'au moins un acide aminotrialkylènephosphonique étant par ailleurs ajoutée et
les particules de la poudre composite présentant une taille moyenne de particule d₅₀ de l'ordre de 10 µm à moins de 200 µm.

2. Poudre composite selon la revendication 1, comprenant des particules de carbonate de calcium, susceptible d'être obtenue selon un procédé, consistant à ajouter de l'acide aminotriméthylènephosphonique, de l'acide aminotriéthylènephosphonique, de l'acide aminotripropylènephosphonique et / ou de l'acide aminotributylènephosphonique.

3. Poudre composite selon la revendication 1 ou 2, comprenant des particules de carbonate de calcium, susceptibles d'être obtenues selon un procédé consistant à procéder à l'introduction du dioxyde de carbone ou du mélange gazeux contenant du dioxyde de carbone jusqu'à ce que le mélange réactionnel présente une valeur pH inférieure à 9.

4. Poudre composite selon au moins l'une quelconque des revendications précédentes, comprenant des particules de carbonate de calcium, susceptibles d'être obtenues selon un procédé consistant à procéder à la réaction de la suspension d'hydroxyde de calcium avec le dioxyde de calcium ou avec le mélange contenant du dioxyde de calcium à une température inférieure à 25°C.

5. Poudre composite selon au moins l'une quelconque des revendications précédentes, comprenant des particules de carbonate de calcium, susceptibles d'être obtenues selon un procédé consistant à diriger du dioxyde de carbone ou du mélange gazeux contenant du dioxyde de carbone avec un débit de gaz dans l'ordre de 0,02 1 CO₂/ (h*g Ca(OH)₂) à 2,0 1 de CO₂ / (h*g Ca(OH)₂) dans la suspension d'hydroxyde de calcium.

6. Poudre composite selon au moins l'une quelconque des revendications précédentes, **caractérisée en ce que** les particules de carbonate de calcium de forme sphérique présentent un diamètre moyen inférieur à 30,0 µm, notamment inférieur à 20,0 µm.

7. Poudre composite selon au moins l'une quelconque des revendications précédentes, **caractérisée en ce que** les particules de carbonate de calcium de forme sphérique présentent un distribution granulométrique dans laquelle au moins 90,0 % en poids de toutes les particules de carbonate de calcium présentent un diamètre de particule de l'ordre compris entre le diamètre moyen de particule - 30 % et le diamètre moyen de particule + 30%.

8. Poudre composite selon au moins l'une quelconque des revendications précédentes, **caractérisée en ce que** les particules de carbonate de calcium de forme sphérique présentent un facteur de forme, défini comme étant le quotient du diamètre minimum de particule et du diamètre maximum de particule, supérieur à 0,90.

9. Poudre composite selon au moins l'une quelconque des revendications précédentes, **caractérisée en ce que** les particules de grande taille comprennent au moins un polymère thermoplastique.

10. Poudre composite selon au moins l'une quelconque des revendications précédentes, **caractérisée en ce que** les particules de grande taille comprennent au moins un polymère résorbable.

11. Poudre composite selon la revendication 10, **caractérisée en ce que** le polymère résorbable fait preuve d'une viscosité inhérente, mesurée dans du chloroforme à 25 °C, à une concentration de polymère de 0,1 % de l'ordre de 0,3 dl/g à 8,0 dl/g.

12. Poudre composite selon au moins l'une quelconque des revendications précédentes, **caractérisée en ce que** les particules de grande taille comprennent de l'acide lactique poly-D, poly-L et / ou poly-D,L.

13. Poudre composite selon au moins l'une quelconque des revendications précédentes, **caractérisée en ce que** les particules de grande taille comprennent au moins un polyester résorbable présentant une masse moléculaire moyenne en nombre de l'ordre compris entre 500 g/mole et 1.000.000 g/mole.

14. Poudre composite selon au moins l'une quelconque des revendications précédentes, **caractérisée en ce que** les particules de grande taille comprennent au moins un polyamide.

15. Poudre composite selon au moins l'une quelconque des revendications précédentes, **caractérisée en ce que** les particules de grande taille comprennent au moins un polyuréthane.

16. Poudre composite selon au moins l'une quelconque des revendications précédentes, **caractérisée en ce que** la part en poids des particules de carbonate de calcium précipitées s'élève à au moins 0,1 % en poids, en rapport au poids total de la poudre composite.

17. Poudre composite selon au moins l'une quelconque des revendications précédentes, **caractérisée en ce que** la poudre composite comprend, en rapport au poids total de la poudre composite de 40,0 % en poids à 80,0 % en poids de PLLA et de 20,0 % en poids à 60,0 % en poids de particules de carbonate de calcium précipitées.

18. Utilisation d'une poudre composite selon au moins l'une quelconque des revendications précédentes en tant qu'additif, notamment d'additif pour polymère, en tant qu'adjuvant ou produit de départ pour le compoundage, pour la fabrication de composants pour des applications en technique médicale et / ou en microtechnologie et / ou pour la fabrication d'objets expansés.

19. Composant, susceptible d'être obtenu par frittage sélectif au laser d'une composition, comprenant une poudre composite selon au moins l'une quelconque des revendications 1 à 17, à l'exception des implants destinés à être utilisés dans le domaine de la neurochirurgie, de la chirurgie buccale, de la chirurgie maxillaire, de la chirurgie faciale, de la chirurgie oto-rhino-laryngologique, ainsi que de la chirurgie de la main, de la chirurgie du pied, de la chirurgie thoracique, de la chirurgie costale et de la chirurgie de l'épaule.

20. Particules de carbonate de calcium de forme sphérique, susceptibles d'être obtenues selon une procédé consistant à
a. prendre une suspension d'hydroxyde de calcium,
b. introduire dans la suspension de l'étape a. du dioxyde de carbone ou un mélange gazeux contenant du dioxyde de carbone et
c. séparer les particules de carbonate de calcium obtenues,
une quantité de 0,3 % en poids à 0,7 % en poids d'au moins un acide aminotrialkylènephosphonique étant par ailleurs ajoutée.

21. Utilisation des particules de carbonate de calcium de forme sphérique selon la revendication 20 en tant qu'additif pour du papier, des matières plastiques, des peintures et / ou des vernis, des élastomères ainsi que des agents adhésifs et des agents d'étanchéité, dans la chimie du bâtiment, dans du mortier sec et dans la technologie médicale, notamment en tant qu'additif dans des polymères résorbables.
